(19) Europäisches Patentamt
European Patent Office
Office européen des brevets

(11) Publication number: **0 135 491**
**B1**

(12) **EUROPEAN PATENT SPECIFICATION**

(45) Date of publication of patent specification: **25.01.89**

(51) Int. Cl.[4]: **A 01 N 43/40,** C 07 D 211/90

(21) Application number: **84870118.1**

(22) Date of filing: **10.08.84**

(54) **Herbicidal 2,6-bis-fluoromethyl-dihydropyridine-3,5-dicarboxylic acid esters.**

(30) Priority: **11.08.83 US 522281**
**24.04.84 US 602022**

(43) Date of publication of application:
**27.03.85 Bulletin 85/13**

(45) Publication of the grant of the patent:
**25.01.89 Bulletin 89/04**

(84) Designated Contracting States:
**AT BE CH DE FR IT LI LU NL SE**

(56) References cited:
**CHEMICAL ABSTRACTS, vol. 88, no. 21, 22 May 1978, Columbus, OH (US); p. 591, no. 152434z**
**JOURNAL OF HETEROCYCLIC CHEMISTRY, vol. 17, no. 5, July 1980, HeteroCorporation, Provo, UT (US); B.SINGH et al.: "Reinvestigation of the reaction of ethyl 4,4,4-trifluoroacetoacetate with aldehydes and ammonia", pp. 1108-1110**

(73) Proprietor: **MONSANTO COMPANY**
**Patent Department 800 North Lindbergh Boulevard**
**St. Louis, Missouri 63166 (US)**

(72) Inventor: **Lee, Len Fang**
**2561 Trenton Station**
**St. Charles, MO 63301 (US)**

(74) Representative: **Lunt, John Cooper et al**
**Monsanto Europe S.A. Patent Department**
**Avenue de Tervuren 270-272 Letter Box No 1**
**B-1150 Brussels (BE)**

**EP 0 135 491 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Background of the Invention

The invention herein pertains to the field of herbicides and more particularly to the field of novel 2 or 6 fluorinated-methyl-dihydropyridine-3,5-dicarboxylic acid esters.

Various dihydropyridines have been known in the art and utilized for various purposes. For example, U.S. Patent 3,969,359 discloses 3,5-dicyano-1,4-dihydropyridines which are useful commercially in scintillation applications as detectors of radioactive radiation.

Dihydropyridines have been utilized for medicinal purposes as exemplified in U.S. Patent 3,441,648 which discloses a large number of 1,4-dihydropyridines. Of particular interest are the 3,5-dicarboxylate compounds having 2,6 substitution. Such compounds are utilized to reduce blood pressure when administered internally. The most active compounds appear to be those substituted at the 4 position by aryl and heterocyclic radicals wherein the hetero atom is either nitrogen, sulfur, or oxygen. Also, trifluoromethylphenyl radicals in the 4 position appear to be highly active in reducing blood pressure in animals.

Of more relevance herein, there is reported by Balicki et al in Polish Patent 89,493, a method for preparing 4-substituted derivatives of 2,6-bis-(trifluoromethyl)-3,5-dicarbethoxy-1,4-dihydropyridines. According to the Polish patent, the compounds have not been previously reported in literature and the compounds so prepared have valuable pharmacological properties, e.g., cytotoxic, bacteriostatic and hypotensive uses as well as intermediate products in the synthesis of new substituted heterocyclic compounds. According to the patent, such compounds are prepared by the reaction of an aldehyde R—CHO in which R represents an alkyl group with 1 to 4 carbons, a substituted or unsubstituted aryl group or a heterocyclic group wherein the hetero atom is nitrogen, oxygen or sulfur with ethyl trifluoroacetoacetate in the presence of a concentrated aqueous solution of ammonia. One molecular equivalent of aldehyde is reacted with two molecular equivalents of the 3-ketoester in aqueous ammonia in the presence of an organic solvent, preferably a lower aliphatic alcohol. The compounds so obtained are identified only by their melting points.

However, it has been further reported in the Journal of Heterocyclic Chemistry, Vol. 17, pages 1109 and 1110 by Singh, et al that the compounds actually prepared by Balicki et al are dihydroxypiperidines rather than dihydropyridines. Singh, et al reports that they have unequivocally shown that products formed by the reaction of ethyl trifluoroacetoacetate with an aromatic aldehyde and a concentrated aqueous solution of ammonia do not have the dihydropyridine structure, but rather are substituted piperidines.

Singh reported that oxidation of the Balicki et al reaction product with nitric acid and several other reagents failed to produce a conversion of the reaction product to the corresponding pyridine. As pointed out in the Singh et al publication, the oxidation procedure is commonly used to convert 1,4-dihydropyridines to the corresponding pyridines, but such conversion did not occur indicating that the reaction product of the process described by Balicki did not provide 1,4-dihydropyridines.

To further substantiate the failure of Balicki et al to teach the preparation of the 2,6-bis-trifluoromethyl-4-substituted-1,4-dihydropyridine-3,5-dicarboxylic acid ester there was provided a series of compounds of structures *1* and *2* of Table I below which were tested to determine their respective melting points. The melting points of compounds 1 and 2 prepared by me were compared to the melting points reported by Balicki et al for the compounds reportedly prepared by them. The results are presented in Table I below.

TABLE I

1

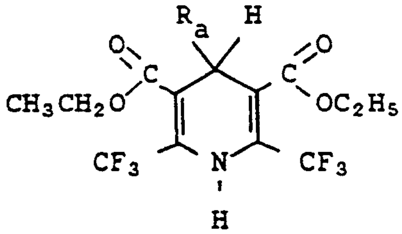

2

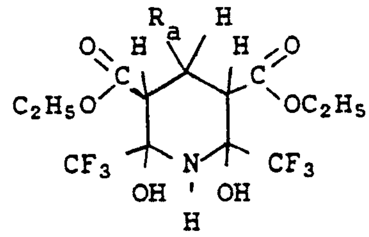

| $R_a$ | Melting Point | | °C |
|---|---|---|---|
| | 1 | 2 | Reported |
| $CH_3$ | 1.4394[a] | 133-135 | 133-134 |
| $C_2H_5$ | 1.4441[a] | 129-131 | 131-132 |
| $C_3H_7$ | 1.4427[a] | 140-142 | 132-133 |
| 2-furyl | 1.4721[a] | 129-130 | 128-129 |
| 2-thienyl | 1.434[a] | 103-105 | -- |
| 4-pyridyl | 171-172 | 179 | 177-178 |
| phenyl | 42-45 | 99-101 | 92-93 |

a $= n_D^{25}$.

The data in Table I provides evidence that the compounds reported by Balicki were, in fact, dihydroxypiperidine and not dihydropyridines. Balicki et al in a later publication (Polish Journal of Chemistry, p. 2439, 1981) corrected the structure assignments of products derived from ethyl trifluoroacetoacetate, aldehydes, and aqueous ammonia as compounds 2.

The dihydroxypiperidines *2* prepared by the method described by Balicki et al consist of a mixture of *cis* and *trans* isomers represented by the structure *A* (*cis* isomer) and *B* (*trans* isomer) as shown below. The structures of the compounds produced by the method of Balicki are further confirmed by X-ray crystallography to be dihydroxypiperidines. The isomeric mixture produced by Balicki's method can be further purified by recrystallization to produce predominantly one isomer. In general, the products presently obtained by the method of Balicki et al have a higher melting point than those reported. This is due to more careful purifications of the isomeric mixture of *2* to provide single *cis* isomer.

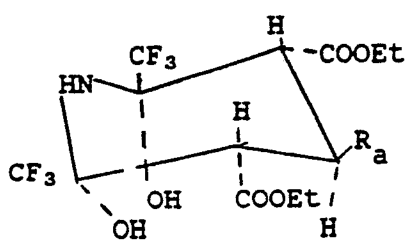

A

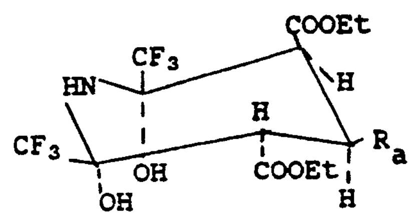

B

In U.S. 4,145,432 there is disclosed 1,4-dihydropyridines useful in pharmaceutical compositions for the treatment of cardiovascular diseases and hypertension in human beings. The active compounds are 3,5-dicarboxylate esters, while the 4-position is occupied by an aryl or heterocyclic group. In addition, the 2 and 6-positions of the dihydropyridine ring are occupied by radicals selected from a wide range of groups including lower alkyl and substituted lower alkyl radicals. Disclosed intermediate compounds include 2,6-halo(lower)alkyl substitution. Also reported in German patent DE 2,659,665 as coronary dilators are 1,4-dihydropyridine derivatives containing a 5-cyano, 3-carboxylate substitution as well as a 2,6-dialkyl substitution. The 4-position of these compounds is occupied by a nitrophenyl radical.

In European patent 44,262 there are reported 1,4-dihydropyridines containing an anilide substituent at the 3-position which are said to be useful as herbicides. However, none of the reported compounds contain substitution at the 4-position or fluoroalkyl groups at the 2- or 6-positions. A substituent at the 5-position is a carboxylic and ester, or nitrile group.

## Summary of the Invention

The present invention relates to herbicidally active compounds, intermediates which provide herbicidally active compounds, a process for preparing the same, herbicidal compositions containing these compounds and herbicidal method of use of said compositions in agricultural crops. The novel compounds of this invention are characterized by the formula *3, 4,* and *5*:

3

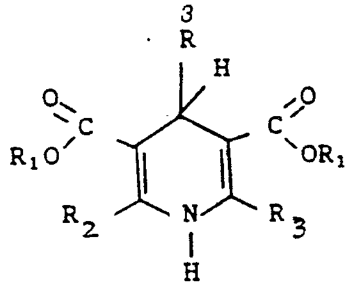

4

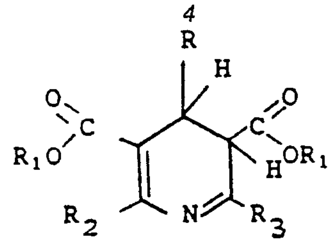

5

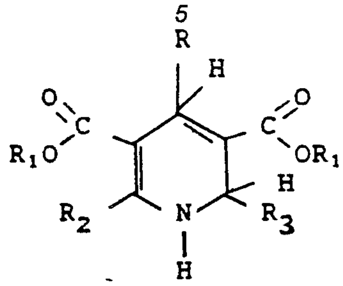

wherein R is selected from the group consisting of phenyl, alkyl, $C_3$—$C_6$ cycloalkyl, haloalkyl, lower alkoxyalkyl, phenylmethoxymethyl, phenoxalkyl, alkylthioalkyl, lower alkylcarbonyloxyalkyl, hydroxyalkyl, cycloalkanylalkyl, and heterocyclic radicals selected from pyridyl, furyl and thienyl radicals, each $R_1$ is independently selected from $C_{1-4}$ alkyl radicals and each $R_2$ is independently selected from fluorinated methyl and $R_3$ is selected from alkyl and fluorinated methyl radicals.

The term "alkyl" means herein both straight and branched chain $C_{1-6}$ alkyl radicals which include, for example, ethyl, methyl, propyl, n-butyl, pentyl, hexyl, isobutyl, isopropyl 1-methylpropyl, neopentyl and 1-ethylpropyl. The term "$C_{3-6}$ cycloalkyl" means cycloalkyl radicals such as cyclopropyl, cyclobutyl, cyclopentyl and cyclohexyl. Typical lower alkoxyalkyl radicals include methoxymethyl, ethoxymethyl and ethoxyethyl. Lower alkylthioalkyl radicals include the thio counterparts of the above-mentioned lower alkoxyalkyl radicals.

The term "haloalkyl" means alkyl $C_{1-6}$ radicals substituted by one or more halogen atoms. Typical examples include fluoromethyl, chloromethyl, difluoromethyl, difluoroethyl, bromomethyl, iodomethyl, dichloromethyl, dichloroethyl, and dibromomethyl, trifluoromethyl, 2-(trifluoromethyl)propyl and chlorofluorinated alkyl radicals.

The term "cycloalkanylalkyl" means alkyl substituted with a cycloalkyl radical.

The term "fluorinated methyl" means methyl radicals having one or more fluorine atoms attached thereto including substitution of all hydrogen atoms by fluorine. This term is intended to include methyl radicals wherein one or more hydrogen atoms may be replaced by a chlorine atom, provided that the methyl radical must have one or more fluorine atoms attached to it. Typical examples include fluoromethyl, difluoromethyl, and trifluoromethyl radicals, chlorodifluoromethyl, dichlorofluoromethyl, and the like.

The term "1,2-dihydropyridine" means herein a dihydropyridine having the hydrogen atoms attached to the ring nitrogen and to an adjacent carbon atom, and includes dihydropyridines which would be called 1,6-dihydropyridines in some chemical nomenclature systems.

The dihydropyridines of this invention of Formula *3* or *4* above are obtained by the dehydration of the corresponding dihydroxypiperidines by any suitable dehydration agent, such as, but not limited to, (1) sulfuric acid; (2) toluensulfonic acid or (3) trifluoroacetic anhydride. Typically the dihydroxypiperidines are obtained from the corresponding tetrahydropyrans by treatment with aqueous or gaseous ammonia. To provide the desired dihydroxypiperidine, the appropriate aldehyde is reacted with an appropriate 3-ketoester and a catalytic amount of piperidine in a suitable reaction medium. This reaction provides the tetrahydropyran from which is obtained the piperidine which, in turn, provides the dihydropyridines by dehydration as mentioned above. The preparation is further described the following reaction scheme:

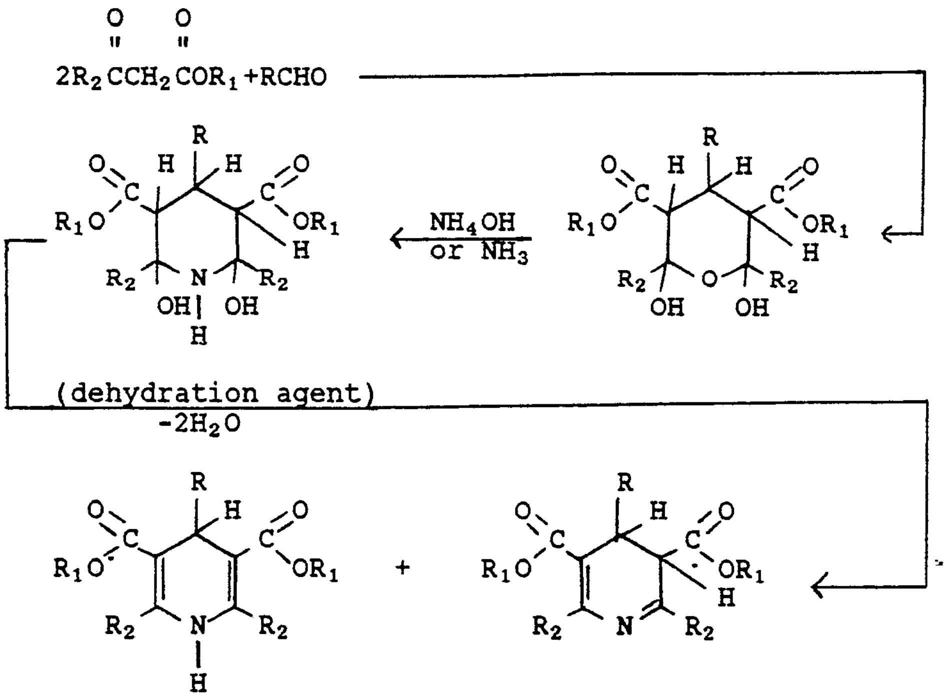


When providing the dihydropyridine compounds wherein the 4-position is substituted by arylmethyl, phenylmethoxymethyl, pyridyl, furyl or thienyl radicals, it is preferred to utilize a catalytic amount of an organic acid as the dehydration agent in place of the usual inorganic acid. Typically, toluenesulfonic acid in a reaction medium of, for example, toluene has been found to be suitable for this purpose. The reaction is generally run at reflux temperature. To provide a major amount of the 3,4-dihydropyridine isomer from the corresponding piperidine, trifluoroacetic anhydride is the preferred dehydration agent. In such process, the reaction medium is typically a chlorinated hydrocarbon such as methylene chloride.

Although the above reaction scheme involves several sequential reactions, in accordance with the process of this invention one can prepare the dihydropyridines in a single reaction mixture. In such a mode of preparation, the reaction product is treated sequentially until the desired product is finally isolated from the reaction mixture as will be further described below. The improved one-vessel reaction provides the desired product in higher yield than is possible in the multi-step process.

In accordance with the novel process of this invention, the appropriate 3-ketoesters represented by the formula

$$R_2-\overset{O}{\overset{\|}{C}}-CH_2-\overset{O}{\overset{\|}{C}}OR_1$$

wherein $R_1$ and $R_2$ are as defined above is first reacted with an aldehyde represented by the formula

$$R-\overset{O}{\overset{\|}{C}}H$$

wherein R is defined above. This reaction occurs under atmospheric pressure and generally at a temperature in the range of from about 40°C to about 100°C. One may optionally employ an aprotic solvent such as methylene chloride or toluene or any suitable solvent for the first reaction. When the reaction of the 3-ketoester and aldehyde is completed, for example, as indicated by NMR analysis, gaseous ammonia or ammonium hydroxide is added through the reaction mixture. If a solvent has not been previously employed it is added prior to the gaseous ammonia. An excess of from about 2 to 10 times the amount of ammonia required for the conversion of the tetrahydropyran is normally employed. After completion of ammonia or ammonium hydroxide addition to the reaction mixture, it is preferred to purge the ammonia from the reaction mixture or drain out excess ammonium hydroxide from the reaction mixture. The purging step is not necessary, but will eliminate the possibility of a reaction of the ammonia with the dehydration agent. The final step of the process is the addition to the reaction mixture of a suitable dehydrating agent whereby the dihydroxypiperidine is converted to the corresponding dihydropyridine. The preferred dehydrating agents in those instances wherein the 4 position of the dihydroxypiperidine is alkyl is sulfuric acid although any suitable dehydrating agent can be used. Sulfuric acid is preferred because it has a greater rate of reaction. Other suitable dehydrating agents are mentioned above. After dehydration of the dihydroxypiperidine to produce the desired dihydropyridine, the product is isolated from the reaction mixture by conventional methods.

The 1,2 dihydropyridines of Formula *5* above are prepared by reduction of the correspondingly-substituted pyridine. The pyridine compounds are formed from the above-described 1,4- and 3,4-dihydropyridines either by oxidation using an oxidizing agent such as sodium nitrite in acetic acid or 2,3-dichloro-5,6-dicyano-1,4-benzoquinone, chromic oxide in acetic acid, or the like when the desired pyridine is to have the same number of fluorine atoms on each $R_2$ as in the starting dihydropyridine. When one of the fluorinated methyl groups is to have one fewer fluorine atoms than the $R_2$ in the parent dihydropyridine, the conversion to the pyridine is carried out by dehydrofluorination using an organic base such as 1,8-diazabicyclo-[5.4.0]-undec-7-ene trialkylamine, pyridine, mono-, di- and tri-alkyl substituted pyridine neat or in a suitable solvent at elevated temperature in the range of 65—160°C.

To form the 1,2 dihydropyridine compounds of Formula *5*, the correspondingly-substituted pyridine is reduced. A preferred reducing agent for this reaction is sodium borohydride in a suitable solvent, such as N,N-dimethylformamide.

Detailed Description of the Invention

The usual starting material in the manufacture of the dihydropyridines of this invention of Formula *3* or *4* above is, as shown above, a dihydroxypiperidine (2). The preferred method for making the aforementioned dihydroxypiperidine involves the reaction of ammonia and the appropriate tetrahydropyran wherein gaseous ammonia is passed through the reaction mixture containing the tetrahydropyran.

The dihydroxypiperidine starting material for the preparation of compounds of this invention can be prepared by either the above-described preferred process or that of the prior art exemplified by Balicki et al in Polish patent 89,493. Utilizing said processes, compounds of the following formula are prepared as listed in Table II below:

TABLE II

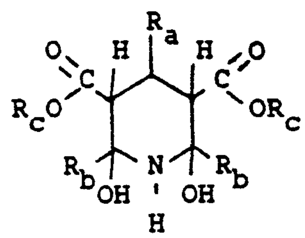

2

| | $R_a$ | $R_b$ | $R_c$ | mp°C |
|---|---|---|---|---|
| A | $CH(CH_3)_2$ | $CF_3$ | $CH_2CH_3$ | 85-89 |
| B | n-butyl | $CF_3$ | $CH_2CH_3$ | 77-80 |
| C | $CH_2CH(CH_3)_2$ | $CF_3$ | $CH_2CH_3$ | 69-73 |
| D | benzyl | $CF_3$ | $CH_2CH_3$ | 137-140 |
| E | $CH_2OCH_2$ 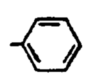 | $CF_3$ | $CH_2CH_3$ | 102-109 |
| F | $CH_2OCH_3$ | $CF_3$ | $CH_2CH_3$ | 122-123 |
| G | $CH_2CH_2OCH_2CH_3$ | $CF_3$ | $CH_2CH_3$ | 1.4269[a] |
| H | $CH_2CH_2SCH_3$ | $CF_3$ | $CH_2CH_3$ | 55-73 |
| I | $CH_2SCH_3$ | $CF_3$ | $CH_2CH_3$ | 102.5-103.5 |
| J | $CH(CH_2CH_3)_2$ | $CF_3$ | $CH_2CH_3$ | 86-89 |
| K | $CH_2CH(CH_3)_2$ | $CF_3$ | $CH_3$ | 102-106 |
| L | $CH_2CH(CH_3)_2$ | $CF_2H$ | $CH_2CH_3$ | 98-100 |

a: $n_D^{25}$

Example 1

Preparation of diethyl 2,6-bis-(trifluoromethyl)-2,6-dihydroxy-4-ethyl-3,5-piperidine dicarboxylate.

A mixture of 368 g (2.0 mol) of ethyl trifluoroacetoacetate, 58 g (1.0 mol) of propionaldehyde and 1 ml of piperidine in 400 ml of $CH_2Cl_2$ is stirred for 1 hour at 20°C, then 1 hour at 50°C and finally is refluxed for 1 hour. An additional 16.0 g (0.289 mol) of propionaldehyde is then added to the above mixture and the mixture is held at reflux for 2 hours after which the heating mantle is removed. To the reaction mixture is passed 108 g (6.35 mol) of ammonia gas in 2 hours. The 19F nmr indicates the reaction mixture contains 77% pure mixture (1:1) of *cis* isomer and *trans* isomer.

The above procedure can be used to prepare other 2,6-dihydroxy 3,5-piperidinedicarboxylates, which are starting materials for preparation of the dihydropyridine compounds of this invention.

Example 2

Preparation of dimethyl 2,6-bis-(trifluoromethyl)-1,4-dihydro-4-isobutyl-3,5-pyridinedicarboxylate and its 3,4-dihydro Isomer.

(a) Preparation from Product of Example K

To an ice cooled mixture of 200 ml. of concentrated sulfuric acid and 200 ml. of methylene chloride is added 48.7 g (0.115 mol) of dimethyl 2,6 - bis - (trifluoromethyl) - 2,6 - dihydroxy - 4 - isobutyl - 3,5 - piperidinedicarboxylate at once. The reaction mixture is stirred for 20 minutes and poured into 1 L. of ice water. The methylene chloride layer is separated, washed once with 100 ml. of saturated sodium bicarbonate, dried and concentrated to provide 28 g (64.6%) of crude product (only 1,4-dihydro isomer). A portion (5.0 g) of this product is kugelrohr distilled at 0.5 torr (pot temperature 120°C) to provide 4.8 g of the desired product as an oil, $n_D^{25}$ 1.4391 which contains a 2:1 mixture of 1,4-dihydro and 3,4-dihydro isomers, respectively.

| | | | |
|---|---|---|---|
| *Anal.* Calc'd. for $C_{15}H_{17}F_6N_1O_4$: | C, 46.28; | H, 4.40; | N, 3.60 |
| Found | C, 46.39; | H, 4.44; | N, 3.60. |

(b) One-vessel Preparation from Methyl Trifluoroacetoacetate

To a mixture of 340 g (2.0 mole) of methyl trifluoroacetoacetate; 100 ml of toluene and 0.86 g of piperidine is added 90.5 g (1.05 moles) of isovaleraldehyde in 20 minutes. The reaction mixture is maintained at 80° for 3 hours and diluted with 125 ml of toluene. Gaseous ammonia (47.3 g, 2.78 moles) is passed in 1.5 hours. The mixture was diluted with 100 ml of toluene. Excess ammonia and 200 ml of toluene are removed under reduced pressure. The reaction mixture is diluted with 100 ml of toluene, cooled to 5°C and treated with 548 g (5.59 moles) of sulfuric acid. The reaction mixture is stirred for 1.5 hours and poured into 2 l of ice water. The toluene layer is separated. The aqueous layer is extracted with 500 ml of toluene. The combined toluene solutions washed successively with 500 ml of water, 500 ml of saturated sodium bicarbonate, and 500 ml of brine, and concentrated *in vacuo* to give 363.6 g of a 59% pure product which corresponds to an 83% overall yield.

Example 3

Preparation of diethyl 2,6-bis-(trifluoromethyl)-1,4-dihydro-4-(4-pyridyl)-3,5-pyridinedicarboxylate.

A single necked 250 ml flask is charged with 100—150 ml concentrated sulfuric acid. The flask is placed in an ice water bath and allowed to stir and cool. To the acid is added 30 g (0.0636 mol) of diethyl 2,6-bis-(trifluoromethyl)-2,6-dihydroxy-4-(4-pyridyl)-3,5-piperidinedicarboxylate and stirring continued for an hour. The acidic mixture is poured over crushed ice and extracted twice with ether. The organics are washed with aqueous saturated sodium bicarbonate, dried on anhydrous magnesium sulfate and stripped of solvent. Five grams of the resulting residue is dissolved in 40 ml of water and again washed with aqueous saturated sodium bicarbonate solution. The organics are extracted with ether and dried on anhydrous magnesium sulfate. The solvent is stripped off and 3.04 g (60.8%) of white powder results. Mp 171—172°C.

| | | | |
|---|---|---|---|
| *Anal.* Calc'd. for $C_{18}H_{16}F_6N_2O_4$: | C, 49.31; | H, 3.65; | N, 6.39 |
| Found | C, 49.33; | H, 3.72; | N, 6.39. |

Example 4

Preparation of diethyl 2,6-bis-(trifluoromethyl)-4-butyl-1,4-dihydro-3,5-pyridinedicarboxylate.

To a 500 ml flask is added 100 ml of concentrated sulfuric acid which is cooled in an ice bath. To the acid is added 4 g (0.0088 mol) of diethyl 2,6 - bis - (trifluoromethyl) - 4 - butyl - 2,6 - dihydroxy - 3,5 - piperidinedicarboxylate. The mixture is stirred for an hour. The acidic mixture is poured over crushed ice and stirred. The organics are extracted with ethyl ether. The ether extract is washed with aqueous saturated sodium bicarbonate solution, dried over $MgSO_4$, and concentrated to yield 2.34 g (63.9%) of product $n_D^{25}$ 1.4419.

| | | | |
|---|---|---|---|
| *Anal.* Calc'd. for $C_{17}H_{21}O_4N_1F_6$: | C, 48.92; | H, 5.03; | N, 3.35 |
| Found | C, 48.95; | H, 5.09; | N. 3.36. |

Example 5

Preparation of diethyl 2,6-bis-(trifluoromethyl)-1,4-dihydro-4-isopropyl-3,5-pyridinedicarboxylate.

To a 500 ml flask is charged 250 ml of concentrated sulfuric acid. The acid is cooled in an ice water bath. To this is added 25 g (0.0569 mol) of diethyl 2,6 - bis - (trifluoromethyl) - 2,6 - dihydroxy - 4 - isopropyl - 3,5 - piperidinedicarboxylate and stirring continued for 90 minutes. The mixture is poured over crushed ice and stirred. The organics are extracted twice with ethyl ether. The ether extract is washed with aqueous saturated sodium bicarbonate solution, dried over $MgSO_4$ and concentrated. The crude material is chromatographed with 10% ethyl acetate/cyclohexane to yield 6.87 g (30%) of product; $n_D^{25}$ 1.444.

*Anal.* Calc'd. for $C_{16}H_{19}O_4N_1F_6$: C, 47.64; H, 4.71; N, 3.47
Found C, 48.95; H, 5.09; N, 3.36.

Example 5

Preparation of diethyl 2,6-bis-(trifluoromethyl)-1,4-dihydro-4-isopropyl-3,5-pyridine-dicarboxylate.

To a 500 ml flask is charged 250 ml of concentrated sulfuric acid. The acid is cooled in an ice water bath. To this is added 25 g (0.0569 mol) of diethyl 2,6 - bis - (trifluoromethyl) - 2,6 - dihydroxy - 4 - isopropyl - 3,5 - piperidinedicarboxylate and stirring continued for 90 minutes. The mixture is poured over crushed ice and stirred. The organics are extracted twice with ethyl ether. The ether extract is washed with aqueous saturated sodium bicarbonate solution, dried over $MgSO^4$ and concentrated. The crude material is chromatographed with 10% ethyl acetate/cyclohexane to yield 6.87 g (30%) of product; $n_D^{25}$ 1.444.

*Anal.* Calc'd. for $C_{16}H_{19}O_4N_1F_6$: C, 47.64; H, 4.71; N, 3.47
Found C, 47.69; H, 4.75; N, 3.46.

Example 6

Preparation of diethyl 2,6-bis-(trifluoromethyl)-1,4-dihydro-4-isobutyl-3,5-pyridinedicarboxylate.

(a) Preparation from Product of Example C

To a 500 ml flask is charged 250—300 ml of concentrated sulfuric acid. The flask is cooled and stirred in an ice water bath. To this mixture is added 28 g (0.0618 mol) of diethyl 2,6 - bis - (trifluoromethyl) - 2,6 - dihydroxy - 4 - isobutyl - 3,5 - piperidinedicarboxylate with stirring. After stirring for an hour the acidic mixture is poured over cracked ice and stirred. The organics are extracted twice with ethyl ether, combined and washed with saturated aqueous sodium bicarbonate, dried over $MgSO_4$ and concentrated. Chromatography with 20% ethyl acetate and cyclohexane yields 9.28 g (36%) of product, $n_D^{25}$ 1.4420.

*Anal.* Calc'd. for $C_{17}H_{21}O_4N_1F_6$: C, 48.92; H, 5.03; N, 3.35
Found C, 48.94; H, 5.08; N, 3.30.

(b) One-vessel Preparation from Ethyl Trifluoroacetoacetate

To a mixture of 368 g (2.0 moles) of etyl trifluoroacetoacetate, 0.9 g of piperidine and 100 ml of toluene is added 90.5 g (1.05 moles) of isovaleraldehyde in 20 minutes. The reaction mixture is maintained at 80°C for 1.5 hours. Additional 4.3 g (0.05 mole) of isovaleraldehyde is added. The reaction mixture is maintained at 80° for 35 minutes and cooled to 50°. To the mixture is passed 30 g (1.76 mole) of gaseous ammonia in 1 hour, then nitrogen gas for 1.5 hours. The reaction mixture is diluted with 200 ml of toluene and cooled to 8°C, then treated with 566 g (5.78 moles) of sulfuric acid. The reaction mixture is stirred at 5—10°C for 45 minutes and poured onto 1.5 kg of ice. The toluene layer is separated, washed successively with 500 ml of brine, 500 ml of saturated aqueous sodium bicarbonate and 500 ml of water, dried ($MgSO_4$) and concentrated to give 394 g of product which is 90% pure and corresponds to an 85% overall yield.

Example 7

Preparation of diethyl 2,6-bis-(trifluoromethyl)-1,4-dihydro-4-propyl-3,5-pyridinedicarboxylate.

Into a 500 ml flask is poured 100—150 ml of sulfuric acid. The flask is placed in an ice bath and the acid is stirred for approximately 15 minutes. Added to acid is 9 g (0.0205 mol) of diethyl 2,6 - bis - (trifluoromethyl) - 2,6 - dihydroxy - 4 - propyl - 3,5 - piperidinedicarboxylate. Stirring continued for 40 minutes. The acidic solution is poured over crushed ice and the resulting aqueous layer is extracted twice with ethyl ether. The organics are washed with saturated aqueous sodium bicarbonate solution, dried on anhydrous magnesium sulfate, filtered, and concentrated to give 2.63 g (31.78%) of product, $n_D^{25}$ 1.4427.

*Anal.* Calc'd. for $C_{16}H_{17}O_4N_1F_6$: C, 47.88; H, 4.23; N, 3.49
Found C, 47.92; H, 4.28; N, 3.47.

Example 8

Preparation of diethyl 2,6-bis-(trifluoromethyl)-1,4-dihydro-4-phenyl-3,5-pyridinedicarboxylate.

A mixture of 1.0 g of diethyl 2,6 - bis - (trifluoromethyl) - 2,6 - dihydroxy - 4 - phenyl - 3,5 - piperidinedicarboxylate, 0.1 g of toluenesulfonic acid and 30 ml of toluene is held at reflux for 4 hours while water is removed azeotropically. The toluene solution is washed with saturated sodium bicarbonate, dried ($MgSO_4$) and concentrated *in vacuo*. The residue is chromatographed on silica gel using ether-petroleum ether (1:9 v/v) as eluant. The first fraction (500 ml of eluate) gives 0.70 g of oil. The $^1H$ nmr analysis of this material shows it is a mixture of 1,4 and 3,4 diethyl 2,6 - bis - (trifluoromethyl) - dihydro - 4 - phenyl - pyridine - 3,5 - dicarboxylate. This material is chromatographed on a silica gel plate (2000 μ, 20 mm × 20

mm) to give 0.66 g (71%) of an oil, $n_D^{25}$ 1.4887 which solidifies to give diethyl 2,6 - bis - (trifluoro - methyl) - 1,4 - dihydro - 4 - phenyl - 3,5-pyridinedicarboxylate mp 42—45°C.

*Anal.* Calc'd. for $C_{19}H_{17}F_6NO_4$: C, 52.18; H, 3.92; N, 3.20
Found C, 52.33; H, 3.95; N, 3.20.

Example 9

Preparation of diethyl 2,6-bis-(trifluoromethyl)-1,4-dihydro-4-(4-pyridyl)-3,5-pyridinedicarboxylate sulfuric acid salt.

A single necked 250 ml flask is charged with 100—150 ml of concentrated sulfuric acid and the acid is cooled in an ice water bath. To this is added 30 g (0.0636 mol) of diethyl 2,6 - trifluoromethyl - 2,6 - dihydroxy - 4 - (4 - pyridyl) - 3,5 - piperidinecarboxylate. The stirring is continued for an hour. The acidic solution is poured over crushed ice and is stirred before being extracted twice with ether. The crystals formed in the aqueous layer are filtered and recrystallized from acetone, mp 140—142°C.

*Anal.* Calc'd. for $C_{18}H_{18}O_8N_2S_1F_6$: C, 40.29; H, 3.35; N, 5.22; S, 5.97
Found C, 40.27; H, 3.38; N, 5.18; S, 5.98.

Example 10

Preparation of diethyl 2,6-bis-(trifluoromethyl)-1,4-dihydro-4-methyl-3,5-pyridinedicarboxylate.

A 200 ml single necked flask is charged with 60 ml of concentrated sulfuric and is placed in an ice water bath to cool. To the acid is added 5 g (0.123 mol) of diethyl 2,6 - bis - (trifluoromethyl) - 2,6 - dihydroxy - 4 - methyl - 3,5 - piperidinedicarboxylate. Stirring continues for 15 minutes and the acidic solution is poured over crushed ice. The solution is extracted twice with ether and then washed with aqueous saturated sodium bicarbonate solution. The organics are dried on anhydrous magnesium sulfate and stripped of solvent. The resulting semi-solid is triturated with petroleum ether and filtered. The filtrate is concentrated to give 0.9 g of product, $n_D^{25}$ 1.4377.

*Anal.* Calc'd. for $C_{14}H_{15}O_4N_1F_6$: C, 44.81; H, 4.03; N, 3.73
Found C, 44.98; H, 4.06; N, 3.67.

Example 11

Preparation of diethyl 2,6-bis-(trifluoromethyl)-1,4-dihydro-4-(phenylmethyl)-3,5-pyridinedicarboxylate.

Toluene (250 ml) is refluxed with a Dean/Stark trap to remove water. In the cooled toluene is added 15 g (0.0308 mol) of diethyl 2,6 - bis - (trifluoromethyl) - 2,6 - dihydroxy - 4 - phenylmethyl - 3,5 - piperidinedicarboxylate and 2 g (0.0105 mol) of toluenesulfonic acid. The solution is heated to reflux and refluxed for 2 hours. An additional 2 g (0.0105 mol) of *p*-toluenesulfonic acid is added and the mixture refluxed for 18 hours while water is removed by means of a Dean/Stark trap.

The mixture is cooled, filtered and concentrated. Ethyl ether is added to the concentrate and the organics are washed with saturated aqueous sodium bicarbonate, separated, and dried over $MgSO_4$ and concentrated.

The residue is chromatographed on silica gel using 10% ethyl acetate/cyclohexane as eluant to yield 1 g (7.2%) of the desired product; $n_D^{25}$ 1.4820.

*Anal.* Calc'd. for $C_{20}H_{21}O_4N_1F_6$: C, 52.98; H, 4.63; N, 3.09
Found C, 53.24; H, 4.27; N, 3.09.

Example 12

Preparation of diethyl 2,6-bis-(trifluoromethyl)-1,4-dihydro-4-(2-furyl)-3,5-pyridinedicarboxylate.

A 250 ml single necked flask is charged with 150 ml toluene. The toluene is refluxed to remove water using a Dean/Stark trap. To the cooled toluene is added 10 g (0.0217 mol) of diethyl 2,6 - bis - (trifluoromethyl) - 2,6 - dihydroxy - 4 - (2 - furyl) - 3, 5-piperidinedicarboxylate and 1 g (0.005 mol) of *p*-toluenesulfonic acid. The reaction mixture is heated to reflux and refluxed for 4 hours while water is removed by a Dean/Stark trap. The toluene is distilled off and the crude product is chromatographed on silica gel using 20% ethyl acetatecyclohexane to give 2.48 g (26.8%) of product, $n_D^{25}$ 1.4720.

*Anal.* Calc'd. for $C_{17}H_{15}F_6N_1O_5$: C, 47.77; H, 3.51; N, 3.27
Found C, 47.83; H, 3.51; N, 3.25.

Example 13

Preparation of diethyl 2,6-bis-(trifluoromethyl)-1,4-dihydro-4-(2-thienyl)-3,5-pyridinedicarboxylate.

Approximately 100 ml of toluene is refluxed using a Dean/Stark trap to remove water. To the cooled toluene is added 20 g (0.0418 mol) of diethyl 2,6 - bis - (trifluoromethyl) - 2,6 - dihydroxy - 4 - (2 - thienyl) - 3,5 - piperidinedicarboxylate and 2.0 g (0.0105 mol) of *p*-toluenesulfonic acid. The mixture is heated to reflux and refluxed for 5-1/2 hours. The solution is cooled and filtered. The solvent is stripped off and the product is chromatographed using 20% ethyl acetate/cyclohexane as eluant to provide 2.45 g (13.23%) of product, $n_D^{25}$ 1.4937.

*Anal.* Calc'd. for $C_{17}H_{15}O_4N_1F_6S_1$: C, 46.04; H, 3.38; N, 3.16; S, 7.22.
Found C, 46.11; H, 3.44; N, 3.12; S. 7.16.

Example 14

Preparation of diethyl 4-methylthiomethyl-2,6-bis-(trifluoromethyl)-1,4-dihydropyridine 3,5-dicarboxylate and diethyl 4-methylthiomethyl-2,6-bis-(trifluoromethyl)-3,4-dihydropyridine 3,5-dicarboxylate.

To a stirred mixture of 37.0 g (0.41 mol) methylthioacetaldehyde and 168 g (0.90 mol) ethyl trifluoroacetoacetate, is added 1.0 ml (0.01 mol) piperidine. The reaction mixture is heated to 80°C resulting in an exotherm, causing the temperature to rise to 105°C. After cooling to 90°C, the reaction mixture is held at this temperature for 2 hours. The cooled intermediate is stripped, dissolved in 200 ml tetrahydrofuran and excess ammonia bubbled into the solution. An exotherm occurs raising the reaction temperature to 40°C. After the exotherm peaked, the reaction temperature is increased to 60°C and held at this temperature for 4 hours. The intermediate is stripped to give 180.3 g of an oil.

A 20 g aliquot of this intermediate in 50 ml methylene chloride is added to an ice water cooled and stirred solution of 20 ml concentrated sulfuric acid in 100 ml methylene chloride at a reaction temperature of less than 10°C. Stirring of the two phase reaction mixture is continued for 5 minutes after addition is complete. The reaction mixture is poured into a 600 ml ice/water mixture. After mixing well, the phases are separated and the aqueous phase extracted with 50 ml methylene chloride. The combined methylene chloride phases are washed successively with 600 ml of 1% sodium chloride solution, and 600 ml of a solution of 1% sodium chloride and 5% sodium bicarbonate, dried over magnesium sulfate, filtered and stripped. Kugelrohr distillation of the liquid residue gives 11.8 g of an oil, collected at 130—150°C/0.4 torr.

Purification of the product by HPLC on silica gel using 10% ethyl acetate in cyclohexane for elution solvent yields a yellow oil after removal of the solvent. Kugelrohr distillation gives 4.41 g of a yellow oil, boiling range 125—135°C/0.08 torr, 23% yield, $n_D^{25}$ 1.4686.

This material contains 89% of the 1,4-dihydropyridine isomer and 11% of 3,4-dihydropyridine isomer.

| | | | |
|---|---|---|---|
| *Anal.* Calc'd. for $C_{15}H_{17}F_6NO_4S$: | C, 42.76; | H, 4.07; | N, 3.32 |
| Found | C, 42.72; | H, 4.24; | N, 3.07. |

Example 15

Preparation of diethyl 2,6-bis-(trifluoromethyl)-1,4-dihydro-4-phenylmethoxymethyl-3,5-pyridinedicarboxylate.

A mixture of 8.6 g (0.0166 mol) of diethyl 2,6 - bis - (trifluoromethyl) - 2,6 - dihydroxy - 4 - phenylmethoxymethyl - 3,5 - piperidinedicarboxylate, 50 ml of toluene and 1.5 g of p-toluenesulfonic acid monohydrate is held at reflux for 19 hours while water is removed by azeotropic distillation. The toluene solution is washed successively with 100 ml of water and 100 ml of saturated sodium bicarbonate, dried ($MgSO_4$) and concentrated. The residue is kugelrohr distilled at 2 mm. The first fraction (pot temperature 110—125°C) is discarded. The second fraction (pot temperature 130—140°C) provides 3.4 g of an oil which is chromatographed using Waters prep 500—A silica gel column with 5% ethyl acetatecyclohexane as eluant (flow rate 250 ml/min). The first fraction (retention time 5—9 minutes) is 2.01 g (25%) of desired product, $n_D^{25}$ 1.4845.

| | | | |
|---|---|---|---|
| *Anal.* Calc'd. for $C_{21}H_{21}F_6N_1O_5$: | C, 52.40; | H, 4.40; | N, 2.91 |
| Found | C, 52.48; | H, 4.42; | N, 2.92. |

Example 16

(a)

Preparation of diethyl 2,6-bis-(trifluoromethyl)-1,4-dihydro-4-ethyl-3,5-pyridinedicarboxylate.

Into a 250 ml single necked flask is poured 100—120 ml of concentrated sulfuric acid. The acid is cooled in an ice/water bath and stirred . To this is added 2.5 g (0.0058 mol) of diethyl 2,6 - bis - (trifluoromethyl) - 4 - ethyl - 1,4 - dihydroxy - 3,5 - piperidinedicarboxylate (*cis* isomer only) and the solution is stirred for 15 minutes. The acidic solution is poured over crushed ice and extracted with ethyl ether. The total organics are washed with saturated sodium bicarbonate, dried and concentrated. The crude product is chromatographed on silica gel using 20% ethyl acetate-cyclohexane to yield 1 g (44.2%) of product based on dihydroxypiperidine of product, $n_D^{25}$ 1.4441.

| | | | |
|---|---|---|---|
| *Anal.* Calc'd. for $C_{15}H_{17}F_6N_1O_4$: | C, 46.27; | H, 4.37; | N, 3.59 |
| Found | C, 46.41; | H, 4.19; | N, 3.62. |

(b)

(Improved Synthesis)

To a well stirred mixture of 200 ml of concentrated sulfuric acid and 200 ml of methylene chloride is added 88.5 g (0.208 mol) of the dihydroxy piperidinedicarboxylate (*cis* isomer only) of (a) above. The reaction mixture is stirred for 45 minutes and poured slowly onto 600 g of cracked ice. The methylene chloride layer is separated, dried ($MgSO_4$) and is concentrated to give 76.7 g (94.7% based on dihydroxypiperidine) of product.

(c)

As mentioned above, one may prepare the 1,4-dihydropyridines of this invention directly from the reaction of an appropriate 3-ketoester and aldehyde. In this example there is described such a procedure.

A mixture of 368 g (2.0 mols) of ethyl trifluoroacetoacetate, 58 g (1.0 mol) of propionaldehyde and 1 ml

of piperidine in 400 ml of methylene chloride is stirred for 1 hour at 20°C then for 1 hour at 30°C and then refluxed for 1 hour and cooled. An additional 16.8 g (0.289 mol) of propionaldehyde is added to the above mixture and the refluxing is continued for 2 hours. The heating mantle is then removed. To the reaction mixture is passed 108 g (6.35 mol) of ammonia gas in 2 hours. The reaction mixture is stirred for 40 hours at 20°C then cooled in an ice water bath. To the cooled reaction mixture is added carefully 100 ml of concentrated sulfuric acid in 20 minutes followed by additional 300 ml of concentrated sulfuric acid in 10 minutes. The reaction mixture is poured onto 600 g of cracked ice in a 4 liter beaker. The methylene chloride layer is separated, dried ($MgSO_4$) and concentrated to give 386 g of an oil which contained a mixture of the desired product and its 3,4-dihydro isomer. This oil is added to a vigorously stirred mixture of 300 ml of concentrated sulfuric acid and 300 ml of methylene chloride. The mixture is stirred for 30 minutes and poured onto 1 kg of ice. The methylene chloride layer is separated, dried ($MgSO_4$) and concentrated to give 348 g of an oil which is triturated with 400 ml of petroleum ether to remove 9.5 g of an insoluble solid. The petroleum ether filtrate is concentrated. The residue is kugelrohr distilled at 0.4 torr to give 290 g (74.5% based on ethyl trifluoroacetoacetate) of an oil which is a mixture of desired product (84%) and its 3,4-dihydro isomer (16%) determined by a $^{19}F$ nmr analysis.

Example 17

Preparation of diethyl 2,6-bis-(trifluoromethyl)-3,4-dihydro-4-hydroxymethyl-3,5-pyridinedicarboxylate.

A mixture of 9 g (0.0174 mol) of *cis* diethyl 2,6 - bis - (trifluoromethyl) - 2,6 - dihydroxy - 4 - benzyloxymethyl - 3,5 - piperidinedicarboxylate and 50 g (0.238 mol) of trifluoroacetic anhydride is stirred for 40 hours and concentrated. The residue is dissolved in 50 ml of ether. The ether solution is washed with saturated sodium bicarbonate, dried over $MgSO_4$ and concentrated to give 8.7 g of brown oil which contained a 2.7:1 mixture of diethyl 2,6 - bis - (trifluoromethyl) - 3,4 - dihydro - 4 - benzyloxymethyl - 3,5 - pyridinedicarboxylate and diethyl 2,6 - bis - (trifluoromethyl) - 1,4 - dihydro - 4 - benzyloxymethyl - 3,5 - pyridinedicarboxylate.

The above oil is dissolved in 40 ml of $CH_2Cl_2$ and treated with 13.7 g of titanium tetrachloride. The resulting solution is stirred for 1 hour and poured into 50 ml of cold 6N HCl. The mixture is extracted with 50 ml of $CH_2Cl_2$ twice. The combined $CH_2Cl_2$ extracts are dried over $MgSO_4$ and concentrated. The residue is chromatographed on silica gel using 20% ethyl acetate-cyclohexane as eluant.

The earlier fraction is discarded. The second fraction is 3.1 g (45%) of diethyl 2,6 - bis - (trifluoromethyl) - 3,4 - dihydro - 4 - hydroxymethyl - 3,5 - pyridinedicarboxylate as an oil, $n_D^{25}$ 1.4366.

| | | | |
|---|---|---|---|
| *Anal.* Calc'd. for $C_{14}H_{15}F_6N_1O_5$: | C, 42.97; | H, 3.86; | N, 3.58 |
| Found | C, 42.98; | H, 3.90; | N, 3.56. |

Example 18

Preparation of diethyl 2,6-bis-(trifluoromethyl)-3,4-dihydro-4-ethyl-3,5-pyridinedicarboxylate.

A mixture of 10 g (0.0235 mol) of diethyl 2,6 - bis - (trifluoromethyl) - 2,6 - dihydroxy - 4 - ethyl - 3,5 - piperidinedicarboxylate, 29.65 g (0.141 mol) of trifluoroacetic anhydride and 30 ml of $CH_2Cl_2$ is stirred for 2 days and concentrated. The residue is dissolved in ether and washed with saturated sodium bicarbonate, dried over $MgSO_4$ and concentrated to give 2.72 g (30%) of diethyl 2,6 - bis - (trifluoromethyl) - 3,4 - dihydro - 4 - ethyl - 3,5 - pyridinedicarboxylate as an oil, $n_D^{25}$ 1.4186.

| | | | |
|---|---|---|---|
| *Anal.* Calc'd. for $C_{15}H_{17}F_6N_1O_4$: | C, 46.28; | H, 4.40; | N, 3.50 |
| Found | C, 46.12; | H, 4.42; | N, 3.46. |

Other dihydropyridines, prepared from the corresponding piperidines in Table II above are provided by a method similar to that described in Example 16(b). The dihydropyridines provided are described in Table III below. The radicals listed for R, $R_1$, and $R_2$ refer to structures 3 and 4 above.

TABLE III

| Ex. No. | R | $R_2$= $R_3$ | $R_1$ | $n_D^{25}$ | C Calc'd | C Found | H Calc'd | H Found | N Calc'd | N Found | 1.4-Dihydro | 3.4-Dihydro |
|---|---|---|---|---|---|---|---|---|---|---|---|---|
| 19 | $CH_2OCH_3$ | $CF_3$ | $CH_2CH_3$ | 1.4436 | 44.45 | 44.26 | 4.23 | 4.40 | 3.46 | 3.22 | 92 | 8 |
| 20 | $CH_2CH_2OCH_2CH_3$ | $CF_3$ | $CH_2CH_3$ | 1.4419 | 47.12 | 46.96 | 4.88 | 4.69 | 3.23 | 3.02 | 85 | 15 |
| 21 | $CH_2CH_2SCH_3$ | $CF_3$ | $CH_2CH_3$ | 1.4688 | 44.14 | 44.12 | 4.40 | 4.19 | 3.22 | 3.05 | 86 | 14 |
| 22 | $CH(CH_2CH_3)_2$ | $CF_3$ | $CH_3CH_3$ | 1.4433 | 50.11 | 50.15 | 5.37 | 4.99 | 3.25 | 3.22 | 100 | |
| 23 | $CH_2CH(CH_3)_2$ | $CF_2H$ | $CH_2CH_3$ | 1.4716 | 53.54 | 53.38 | 6.08 | 6.40 | 3.67 | 3.25 | 100 | |

Example 24

Preparation of diethyl 2,6-bis-(difluoromethyl)-1,4-dihydro-4-propyl-3,5-pyridinedicarboxylate.

To a stirred mixture of 50.0 g (0.299 mol) of ethyl difluoroacetoacetate and 13.2 mol (0.150 mol) of butyraldehyde is added a few ml of piperidine. The temperature of the reaction mixtures rises spontaneously to 100°C. After the temperature has subsided, the reaction mixture is treated with THF (100 ml) and refluxed for 1.5 hr and allowed to stir at normal temperature for 18 hours. The reaction mixture is then concentrated to give 60.8 g of an oil. Into a solution of 55.8 g (0.138 mol) of above oil in 30 ml of THF is passed $NH_3$. The temperature of the reaction mixture rises spontaneously to 41°C. After the temperature has subsided, the reaction mixture is concentrated to an oil which solidifies upon standing. Recrystallization of this solid from hexane gives 27.6 g (49.7%) of a yellow solid identified as *cis* isomer of diethyl 2,6 - bis - (difluoromethyl) - 2,6 - dihydroxy - 4 - propyl - 3,5 - piperidinecarboxylate. A 5.0 g (0.012 mol) portion of the above solid is stirred with 20 ml of trifluoroacetic anhydride. The reaction temperature rises to 36°C. After the temperature subsides, the reaction mixture is concentrated. The residue is dissolved in ether and washed with saturated sodium bicarbonate, dried ($MgSO_4$) and concentrated to give 3.48 g of an oil which is kugelrohr distilled twice at 0.2 torr (pot temperature 85°C) to give 2.84 g (51.5%) of diethyl 2,6 - bis - (difluoromethyl) - 4 - propyl - 1,4 - dihydro - 3,5 - pyridinedicarboxylate as a yellow oil, $n_D^{25}$ 1.4726.

| | | | |
|---|---|---|---|
| *Anal.* Calc'd. for $C_{16}H_{21}F_4NO_4$ | C, 52.32; | H, 5.76; | N, 3.81 |
| Found | C, 51.98; | H, 5.86; | N, 3.66. |

Example 25

Preparation of diethyl 2,6-bis-(difluoromethyl)-4-cyclohexyl-1,4-dihydro-3,5-pyridinedicarboxylate.

This material is prepared from ethyl difluoroacetoacetate and cyclohexanecarboxaldehyde according to the procedure described in Example 24. The crude product is purified by HPLC using 1% ethyl acetate-cyclohexane as eluant. The earlier fraction is discarded. The second fraction is diethyl 2,6 - bis - (difluoromethyl) - 4 - cyclohexyl - 1,4 - dihydro - 3,5 - pyridinedicarboxylate, mp 40—44°C.

| | | | |
|---|---|---|---|
| *Anal.* Calc'd. for $C_{19}H_{25}F_4NO_4$: | C, 56.02; | H, 6.18; | N, 3.44 |
| Found | C, 56.16; | H, 6.42; | N, 3.42. |

Example 26

Preparation of diethyl 2,6-bis-(trifluoromethyl)-4-cyclohexyl-3,4-dihydro-3,5-pyridinedicarboxylate.

This material is prepared from ethyl trifluoroacetoacetate and cyclohexanecarboxyaldehyde according to the procedure of Example 16(c) except the crude product is not distilled. It is isolated as an oil $n_D^{25}$ 1.4586.

| | | | |
|---|---|---|---|
| *Anal.* Calc'd. for $C_{19}H_{23}F_6N_1O_4$: | C, 51.47; | H, 5.23; | N, 3.16 |
| Found | C, 50.15; | H, 5.38; | N, 3.24. |

Example 27

Preparation of diethyl 2,6-bis-(trifluoromethyl)-1,4-dihydro-4-hydroxymethyl-3,5-pyridinedicarboxylate.

To a solution of 198.5 g (0.385 mol) of the compound as Example E in Table II in 500 ml of $CH_2Cl_2$ is added 268 g (1.28 mol) of trifluoroacetic anhydride. The reaction mixture is stirred for 18 hours and concentrated *in vacuo*. The residue is dissolved in 500 ml of ether and stirred with saturated sodium bicarbonate while 100 g of sodium bicarbonate is added continuously until all residual trifluoroacetic acid has been neutralized. The ether layer is dried ($MgSO_4$) and concentrated to give 178 g of an oil. A solution of the above oil in 500 ml of $CH_2Cl_2$ is cooled to −78°C then treated with 140 g (0.740 mol) of titanium tetrachloride at once. After stirring for 30 minutes at −78°C the reaction mixture is allowed to warm to room temperature then poured into a mixture of 500 ml of concentrated HCl and 500 ml of ice water. The resulting mixture is filtered through Celite. The $CH_2Cl_2$ layer is separated, dried ($MgSO_4$) and concentrated to a mixture of oil and gum. This mixture is treated with 500 ml of ether and filtered to remove gum. The ether filtrate is concentrated to give 149 g of an oil which is chromatographed on silica gel in front portions using 20% ethyl acetate-cyclohexane as eluant. Similar fractions are combined. The earlier fraction (retention time 6—8.5 minutes) is discarded.

The second fraction (retention time 9.5—23 minutes) is crystallized from petroleum ether to give 61 g (42%) of diethyl 2,6 - bis - (trifluoromethyl) - 1,4 - dihydro - 4 - hydroxymethyl - 3,5 - pyridinedicarboxylate as a white solid, mp 65—66°C.

| | | | |
|---|---|---|---|
| *Anal.* Calc'd. for $C_{14}H_{15}F_6N_1O_5$: | C, 42.97; | H, 3.86; | N, 3.58 |
| Found: | C, 42.97; | H, 3.87; | N, 3.58. |

Example 28

Preparation of diethyl 2,6-bis-(trifluoromethyl)-4-chloromethyl-1,4-dihydro-3,5-pyridinedicarboxylate.

A mixture of 2.84 g of triphenylphosphine, 3.91 g (0.01 mol) of the product of Example 27 and 30 ml of $CCl_4$ is held at reflux for 18 hrs., cooled and filtered. The filtrate is concentrated. The residue is heated with hot petroleum ether (100 ml), filtered and concentrated. The residue (3.0 g) is chromatographed on silica gel using $CH_2Cl_2$ as eluant. The earlier fraction (1.5L of eluate) gives 2.0 g of an oil which is rechromatographed on silica gel using 5% ethyl acetate-petroleum ether as eluant to give three fractions.

The first fraction (retention time 6—8 minutes) is 0.3 g (7.3%) of diethyl 2,6 - bis - (trifluoromethyl) - 4 - chloromethyl - 1,4 - dihydro - 3,5 - pyridinedicarboxylate as an oil, $n_D^{25}$ 1.4592.

| | | | | |
|---|---|---|---|---|
| *Anal.* Calc'd. for $C_{14}H_{14}Cl_1F_6N_1O_4$: | C, 41.04; | H, 3.44; | N, 3.42; | Cl, 8.65 |
| Found | C, 41.02; | H, 3.47; | N, 3.39; | Cl, 8.65. |

Example 29

Preparation of diethyl 4-acetoxymethyl-2,6-bis-(trifluoromethyl)-1,4-dihydro-3,5-pyridinedicarboxylate.

A solution of 3.91 g (0.01 mol) of the product of Example 27 in 20 ml acetic anhydride and 5.0 g (0.637 mol) of acetyl chloride is stirred for 1 hour and concentrated to give an oil which crystallized. The resulting solid is recrystallized from 50 ml of petroleum ether (30—75°C) to give 3.84 g (88.7%) of diethyl 4 - acetoxymethyl - 2,6 - bis - (trifluoromethyl) - 1,4 - dihydro - 3,5 - pyridinedicarboxylate, mp 101—103°C.

| | | | |
|---|---|---|---|
| *Anal.* Calc'd. for $C_{16}H_{17}F_6N_1O_6$: | C, 44.35; | H, 3.96; | N, 3.23 |
| Found | C, 44.19; | H, 3.98; | N, 3.18. |

Example 30

Preparation of diethyl 2,6-bis-(trifluoromethyl)-1,4-dihydro-4-neopentyl-3,5-pyridinedicarboxylate and its 3,4-dihydro isomer.

A mixture of 62 g (0.50 mol) of 78% pure 3,3-dimethylbutyraldehyde, 184 g (1.0 mol) of ethyl trifluoroacetoacetate, 1 ml of piperidine and 300 ml of THF is held at reflux for 32 hours and cooled to 50°C. To the above solution is passed 100 g (6.47 mol) of ammonia in 10 hours. The reaction mixture is concentrated. The residue is dissolved in 500 ml of ether. The solution is washed twice with 300 ml of water, dried ($MgSO_4$) and concentrated. The residue (179 g) is added slowly in 15 minutes to an ice cooled, mechanically stirred mixture of 400 ml of concentrated sulfuric acid and 400 ml of $CH_2Cl_2$. The mixture is poured onto 2 kg of crushed ice and stirred well. The $C_2Cl_2$ layer is separated, dried ($MgSO_4$) and concentrated. The residue is kugelrohr distilled at 0.5 torr. The earlier fraction is discarded. The second fraction (pot temperature 91—130°C) is 122 g of a liquid which is fractionally distilled at 1 torr. The fraction with bp of 121—130°C is pure desired product as a yellow oil $n_D^{25}$ 1.4388 which contains a 3:1 mixture of 1,4-dihydro and 3,4-dihydro isomers.

| | | | |
|---|---|---|---|
| *Anal.* Calc'd. for $C_{18}H_{23}F_6N_1O_4$: | C, 50.12; | H, 5.37; | N, 3.25 |
| Found | C, 49.92; | H, 5.25; | N, 3.65. |

Example 31

Preparation of diethyl 2,6-bis-(trifluoromethyl)-1,4-dihydro-4-(1-methylpropyl)-3,5-pyridinecarboxylate and its 3,4-dihydro isomer.

This material is prepared from 2-methylbutaldehyde and ethyl trifluoroacetoacetate by a procedure similar to that used in Example 30. It is obtained as a colorless oil, $n_D^{25}$ 1.4417.

| | | | |
|---|---|---|---|
| *Anal.* Calc'd. for $C_{17}H_{21}F_6N_1O_4$: | C, 48.92; | H, 5.07; | N, 3.36 |
| Found | C, 49.05; | H, 5.10; | N, 3.34. |

With the exception of the products of Examples 17 and 27, the above described dihydropyridines may be utilized as intermediates in a method for providing herbicidally active corresponding pyridines. The conversion of the above described dihydrpyridines to corresponding pyridines is carried out by reacting the dihydropyridines with 1,8-diazobicyclo-[5.4.0]-undec-7-ene (DBU) in refluxing tetrahydrofuran. The procedure in which the dihydropyridines can be utilized as intermediates for the production of herbicidally active pyridines is exemplified by the following examples.

Example 32

Preparation of diethyl 2-(difluoromethyl)-4-propyl-6-(trifluoromethyl)-3,5-pyridinedicarboxylate.

A mixture of 31.0 g (0.0742 mol) of the product of Example 7, 11.03 g (0.0742 mol) of DBU and 200 ml of THF is held at reflux for 18 hours and concentrated. The residue is stirred with water and extracted with ether. The ether extracts ae dried ($MgSO_4$) and concentrated. The residue is kugelrohr distilled at 1 torr. The distillate is chromatographed on silica gel using 3% ethyl acetatecyclohexane as eluant to give 23.9 g (80.9%) of diethyl 2 - (difluoromethyl) - 4 - propyl - 6 - (trifluoromethyl) - 3,5 - pyridinedicarboxylate as an oil $n_D^{25}$ 1.4436.

| | | | |
|---|---|---|---|
| *Anal.* Calc'd. for $C_{16}H_{18}F_5NO_4$: | C, 50.13; | H, 4.74; | N, 3.66 |
| Found | C, 49.74; | H, 4.66; | N, 3.55. |

Example 33

Preparation of diethyl 4-cyclohexyl-2-(difluoromethyl)-6-(trifluoromethyl)-3,5-pyridinedicarboxylate.

A mixture of 70.0 g (0.152 mol) of the product of Example 26, 23.15 (0.152 mol) of DBU and 250 ml of THF is held at reflux for 18 hours and concentrated. The residue is poured into water and extracted with ether. The ether extracts are washed with diluted hydrochloric acid, dried ($MgSO_4$) and concentrated. The residue is kugelhohr distilled to give 31.9 g (49.4%) of diethyl 4 - cyclohexyl - 2 - (difluoromethyl) - 6 - (trifluoromethyl) - 3,5 - pyridinedicarboxylate as an oil $n_D^{25}$ 1.4614.

| | | | |
|---|---|---|---|
| *Anal.* Calc'd. for $C_{19}H_{22}F_5N_1O_4$: | C, 53.90; | H, 5.24; | N, 3.31 |
| Found | C, 54.19; | H, 5.33; | N, 3.51. |

Example 34

Preparation of dimethyl 2-(difluoromethyl)-6-(trifluoromethyl)-4-isobutyl-3,5-pyridinedicarboxylate

A mixture of 23.0 g (0.0591 mol) of the product of Example 2, 12.2 g (0.077 mol) of 96% pure DBU, and 100 ml of THF is held at reflux for 3 days and poured into 250 ml of 3 N HCl. The oil precipitate is extracted into ether (2 × 100 ml). The ether extracts are dried ($MgSO_4$) and concentrated to give 14.4 g of an oil which, according to $^1H$ NMR, contained the desired product and acidic products. This oil is dissolved in ether and extracted with 100 ml of saturated sodium bicarbonate. The ether layer is dried ($MgSO_4$) and concentrated to give 8.9 g of an oil which is 71% pure desired product (by $^{19}F$ NMR).

The sodium bicarbonate extract is acidified with concentrated HCl to give an oil which is extracted into ether. The ether layer is dried ($MgSO_4$) and concentrated to give 4.8 g of a residue which contained monocarboxylic acid and dicarboxylic acid (9:1) derived from the desired product. This residue is treated with 3.0 g (0.0217 mol) of potassium carbonate, 20 ml of methyl iodide, and 50 ml of acetone. The mixture is held at reflux for 42 hours and concentrated. The residue is treated with water and extracted with ether (2 × 100 ml). The ether layer is dried and concentrated. The residue is kugelrohr distilled at 1 torr (pot temperature of 130°C) to give 5.1 g (23.4% from Example 2) of the desired product as an oil, $n_D^{25}$ 1.4478. This product crystallized after standing, m.p. 36—37°C.

*Anal.* Calc'd. for $C_{15}H_{16}F_5N_1O_4$: C, 48.79; H, 4.37; N, 3.79
Found: C, 48.75; H, 4.39; N, 3.77.

The 71% pure desired product described previously was chromatographed by HPLC using 3% ethyl acetate/cyclohexane as eluent to give an earlier fraction (0.79 g, retention time 7—8.5 min.) which was identified as methyl 6 - (difluoromethyl) - 4 - (isobutyl) - 2 - (trifluoromethyl) - 3 - pyridinecarboxylate. The second fraction (retention time 8.5—18.5 min.) is an additional 6.4 g (29.4%) of pure desired product, $n_D^{25}$ 1.4474.

As has been previously stated, 1,2-dihydropyridines of this invention are prepared by reduction of the correspondingly-substituted pyridine compound with a reducing agent such as sodium borohydride. This procedure is illustrated in the following Examples 35—37.

Example 35

Preparation of dimethyl 1,2-dihydro-2-(difluoromethyl)-4-isobutyl-6-(trifluoromethyl)-3,5-pyridinedicarboxylate

To a solution of 21.9 g (0.06 mol) of product of Example 34 in 110 ml of DMF was added successively 4.6 g (0.121 mol) of sodium borohydride and 30 ml of water. After 2 hours of stirring additional 4.6 g (0.121 mol) of sodium borohydride, 30 ml of water and 100 ml of DMF were added in succession, and the reaction mixture was stirred for 4 hours and concentrated. The crude product was purified by HPLC using 5% ethyl acetate in cyclohexane as eluent. The first fraction was 3.9 g of an oil containing a mixture of unidentified products. The second fraction (0.6 g) was not characterized. The third fraction was 6.2 g of an oil which was crystallized from hexane to give 2.3 g (10%) of yellow solid. Recrystallization twice from hexane yielded 1.34 g (6%) of desired product as a yellow solid, mp 87.5—88.5°C.

*Anal.* Calc'd. for $C_{15}H_{18}F_5NO_4$: C, 48.52; H, 4.89; N, 3.77
Found: C, 48.54; H, 4.68; N, 3.83.

Example 36

Preparation of dimethyl 1,2-dihydro-6-(difluoromethyl)-4-isobutyl-2-(trifluoromethyl)-3,5-pyridinedicarboxylate

A solution of 53.0 g (0.144 mol) of product of Example 34 was reduced with 25 g (0.661 mol) of sodium borohydride as described in Example 35. The crude product (64.4 g) was purified by HPLC using 5% ethyl acetate in cyclohexane as eluent. The first fraction (7.9 g) was unidentified product (27%). The second fraction (1.4 g) was not characterized. The third fraction was 29.9 g of oil containing desired product. Crystallization of this oil from hexane gave 26.9 g of a first crop, mp 76—82°C, 5.0 g of a second crop, and 1.1 g of a third crop. The first crop was a mixture of product of Example 35 and desired product in a ratio of 2.7:1. The second crop and the third crop were combined and recrystallized six times from cyclohexane to give 0.7 g of pure product, as yellow solids, mp 79—81°C.

*Anal.* Calc'd. for $C_{15}H_{18}F_5NO_4$: C, 48.52; H, 4.89; N, 3.77
Found: C, 48.37; H, 4.93; N, 3.73.

Example 37

Preparation of dimethyl 1,2-dihydro-4-isobutyl-2,6-bis(trifluoromethyl)-3,5-pyridine dicarboxylate

A 250 ml flask is charged with 35 ml of glacial acetic acid and 13.89 g (0.0354 mol) of product of Example 6. Sodium nitrite is added in the amount of 3 g (0.0434 mol) and the mixture is stirred for 72 hours under nitrogen. The solution is poured over ice/water and stirred. The organics are extracted in ether and washed with aqueous saturated sodium bicarbonate solution. Organics are then dried on anhydrous magnesium sulfate, filtered, and concentrated to yield 4.93 g (35.6%) of diethyl 2,6 - bis(trifluoromethyl) - 4 - isobutyl - 3,5 - pyridinedicarboxylate.

This pyridine compound is hydrolyzed and reesterified to form the dimethyl ester as follows:

A stirred mixture of 31.8 g (0.076 mols) the diethyl ester, 150 ml, 10% sodium hydroxide (.38 mols) and

75 ml ethanol is heated at reflux for 72 hours. The reaction mixture partially concentrated, diluted with water to 250 ml and heated again at reflux for 24 hours. The cooled mixture is acidified with excess concentrated hydrochloric acid, and extracted with ether (200 ml once, 100 ml three times). The combined ether extracts are dried over $MgSO_4$, filtered and concentrated to give 28.5 g residue. Part of this residue (2.2 g) is stirred with 200 ml dimethyl formamide, 16.5 g (0.12 mols) potassium carbonate and 25.5 g (0.18 mols) methyl iodide for 24 hours at room temperature. The mixture is added to 1 liter 2% hydrochloric acid. The product is extracted with methylene chloride (200 ml once, 100 ml three times). The combined extracts are washed with 200 ml 1% hydrochloric acid/1% sodium chloride, dried over $MgSO_4$, filtered and concentrated. Distillation using kugelrohr yields 16.2 g lite yellow oil BP 100—110°C/0.1 torr that solidifies. Recrystallization from hexane/ether affords 12.0 g (49%) white solid m.p. 80.5—82.5°C.

To a stirred solution of 8.65 g (.022 mols) of above solid in 80 ml dimethyl formamide is added 0.83 g (0.22 mols) sodium borohydride. The reaction mixture is stirred at ambient temperature for one hour then warmed to 50°C for 15 minutes and cooled to ambient temperature. The reaction mixture is slowly added to a stirred mixture of 600 ml 2% hydrochloric acid and 100 ml methylene chloride. The phases are separated, the aqueous phase is extracted with 50 ml methylene chloride. The combined extracts are washed with 300ml 1% hydrochloric acid/1% sodium chloride, dried over $MgSO_4$, filtered and concentrated.

Purification by Silica gel HPLC (4% ethyl acetate in cyclohexane) followed by kugelrohr distillation affords 6.79 g (79%) yellow oil BP 115—125°C/0.15 torr, $n_D^{25}$ 1.4584.

| | | | |
|---|---|---|---|
| *Anal.* Calc'd. for $C_{15}H_{17}NO_4F_6$: | C, 46.28; | H, 4.40; | N, 3.60 |
| Found: | C, 46.40; | H, 4.42; | N, 3.36. |

Example 38

3-Ethyl 5-Methyl 1,4-dihydro-4-methyl-2-isopropyl-6-(trifluoromethyl)-3,5-pyridinedicarboxylate

An amount of 52.7 g of ethyl 3-amino-4-methyl-2-pentenoate was prepared from 98 g ethyl (2-methylpropionyl) acetate using the procedure of Aberhart and Liv, JOC *1981*, 3749. The enamino ester had $n_D^{25}$ = 1.4914, bp 92°/6 mm. The above enamine (20 g) was mixed in 100 ml THF with 21.6 g methyl 2,2,2-trifluoroacetoacetate and 6 g of acetaldehyde. Upon adding a couple of drops of piperidine, a spontaneous isotherm to 60° was observed. The mixture was then heated with stirring at about 70° (just below reflux) for 1½ hours. While monitoring with $^{19}F$ nmr, the mixture was refluxed for 5 hours, then allowed to stand at room temperature overnight. The mixture was stripped of THF to give 46.1 g yield of crude product, 40 g of which was dehydrated using 25 ml trifluoroacetic anhydride and about 100 ml $CH_2Cl_2$. An exotherm to 40° was observed, and the material was refluxed for about one hour, then stripped to give 4 g of crude product which was kugelrohr distilled (100—160°/0.15 mm) $n_D^{25}$ = 1.4804.

| | | | |
|---|---|---|---|
| *Anal.* Calc'd. for $C_{15}H_{20}F_3O_4$: | C, 53.75; | H, 6.01; | N, 4.18 |
| Found: | C, 54.20; | H, 6.08; | N, 3.95. |

Example 39

3-Ethyl 5-methyl 1,2-dihydro-2-ethyl-4-isobutyl-6-(trifluoromethyl)-3,5-pyridinecarboxylate

A stirred mixture of 18.0 g (.20 mole) isovaleraldehyde, 30.8 g (.20 mole) methyl trifluoroacetoacetate, 28.6 g (.20 mole) ethyl 3-amino-2-penteneoate, 60 ml tetrahydrofuran and 3 drops piperidine is heated and held at reflux for 18 hours. The cooled reaction mixture is concentrated and the residue partially crystallizes on standing at ambient temperature. The solids are filtered from an 8.0 g sample, washed with hexane and recrystallized from tetrahydrofuran/hexane to give 1.72 g (22%) white solid.

A stirred mixture of 72 g (.18 mole) crude solid prepared as above, 30 ml (.21 mole) trifluoroacetic anhydride and 150 ml methylene chloride is heated and held at reflux for 2 hours. The washed reaction mixture is concentrated to give 88.0 g oil. Purification of a portion by HPLC gives a purified product: $n_D^{25}$ = 1.4785.

| | | | |
|---|---|---|---|
| *Calc'd.* for $C_{16}H_{23}F_3NO_4$: | C, 56.19; | H, 6.66; | N, 3.85 |
| Found: | C, 56.27; | H, 6.77; | N, 3.60. |

Using the preparative techniques similar to those shown in the preceding examples, further compounds of this invention were prepared. These are shown in the following Table A, along with a physical property of each.

TABLE A

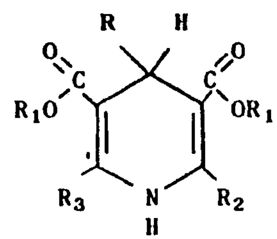

| Ex. No. | R | $R_2$ | $R_3$ | $R_1$ | $R_1'$ | M.P. | BP |
|---|---|---|---|---|---|---|---|
| 40 | 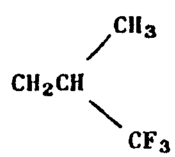 | $CF_3$ | $CF_3$ | Et | Et | | 100/0.5 torr |
| 41 | 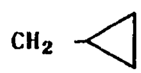 | $CF_3$ | $CF_3$ | Et | Et | | 120/0.5 torr |
| 42 | $CF_3$ | $CF_3$ | $CF_3$ | Et | Et | 60-61 | |
| 43 | $CH_2CH(CH_3)_2$ | $CF_3$ | $CH_3$ | $CH_3$ | $CH_3$ | 68-69 | |
| 44 | $CH_3$ | $CF_3$ | $CH_3$ | Et | Et | | 110-120/0.1 torr |
| 45 | $CH_2CH(CH_3)_2$ | $CF_3$ | $CH_3$ | Et | Et | | 140-145/0.15 torr |
| 46 | $CH_2CH(CH_3)_2$ | $CF_2H$ | $CH_3$ | Et | Et | | 140-150/0.1 torr |

As noted above, the compounds of this invention have been found to be effective as herbicides, particularly as pre-emergent herbicides. Tables IV and V summarize results of tests conducted to determine the pre-emergent herbicidal activity of the compounds of this invention.

The pre-emergent tests are conducted as follows:

A good grade of top soil is placed in aluminum pans and compacted to a depth of 0.95 to 1.27 cm from the top of the pan. On the top of the soil is placed a predetermined number of seeds or vegetative propagules of various plant species. The soil required to level fill the pans after seeding or adding vegetative propagules is weighed into the pan. A known amount of the active ingredient applied to a solvent or as a wettable powder suspension and the soil are thoroughly mixed, and used as a cover layer for prepared pans. In Table IV below the amount of active ingredient is equal to the rate of 11.2 k/ha. After treatment, the pans are moved into a greenhouse bench where they are watered from below as needed to give adequate moisture for germination and growth.

Approximately 2—3 weeks after seeding and treating, the plants are observed and the results recorded. Table IV below summarizes such results. The herbicidal rating is obtained by means of a fixed scale based on the percent injury of each plant species. The ratings are defined as follows:

| % (Inhibition) | Rating |
|---|---|
| 0—24 | 0 |
| 25—49 | 1 |
| 50—74 | 2 |
| 75—100 | 3 |

The plant species utilized in one set of tests, the data for which are shown in Table V, are identified by letter in accordance with the following legend:

| | | |
|---|---|---|
| A-Canada Thistle* | E-Lambsquarters | I-Johnsongrass* |
| B-Cocklebur | F-Smartweed | J-Downy Brome |
| C-Velvetleaf | G-Yellow Nutsedge* | K-Barnyardgrass |
| D-Morningglory | H-Quackgrass* | |

* Grown from vegetative propagules.

TABLE IV

Pre-Emergent

| Compound of Example No. | Plant Species A | B | C | D | E | F | G | H | I | J | K |
|---|---|---|---|---|---|---|---|---|---|---|---|
| 2 | 3 | 2 | 3 | 3 | 3 | - | 3 | 3 | 3 | 3 | 3 |
| 3 | - | 0 | 1 | 0 | 2 | 1 | 0 | 0 | 0 | 2 | 3 |
| 4 | 0 | 0 | 0 | 2 | 3 | 3 | 0 | 0 | 0 | 3 | 3 |
| 5 | - | 0 | 0 | 0 | 0 | 0 | 0 | 0 | 0 | 0 | 1 |
| 6 | - | 1 | 3 | 3 | 3 | 0 | 0 | 3 | 1 | 3 | 3 |
| 7 | 3 | 1 | 3 | 3 | 3 | 3 | 0 | 3 | 3 | 3 | 3 |
| 8 | 0 | 0 | 0 | 1 | 0 | 0 | 0 | 0 | 0 | 0 | 3 |
| 9 | - | 0 | 0 | 0 | 2 | 0 | 0 | 0 | 2 | 0 | 3 |
| 10 | - | 0 | 1 | 3 | 3 | 3 | 0 | 3 | 1 | 3 | 3 |
| 11 | - | 0 | 0 | 0 | 0 | 0 | 0 | 0 | 0 | 0 | 2 |
| 12 | 3 | 0 | 3 | 3 | 3 | 3 | 0 | 1 | 0 | 3 | 3 |
| 13 | 0 | - | 0 | 1 | 3 | 1 | 0 | 0 | 0 | 3 | 3 |

TABLE IV (Cont.)

| Compound of Example No. | Plant Species A | B | C | D | E | F | G | H | I | J | K |
|---|---|---|---|---|---|---|---|---|---|---|---|
| 14 | 0 | 0 | 0 | 2 | 3 | 0 | 0 | 0 | 0 | 3 | 3 |
| 15 | - | 0 | 0 | 0 | 0 | 0 | 0 | 0 | 0 | 0 | 2 |
| 16 | 3 | 1 | 3 | 3 | 3 | - | 0 | 3 | 3 | 3 | 3 |
| 18 | - | 1 | 3 | 3 | 3 | 3 | 1 | 3 | 3 | 3 | 3 |
| 19 | 3 | 0 | 0 | 3 | 3 | 1 | 0 | 2 | 0 | 3 | 3 |
| 20 | 0 | 1 | 1 | 1 | 3 | - | 3 | 3 | 0 | 3 | 3 |
| 21 | 1 | 0 | 0 | 2 | 3 | - | 0 | 0 | 0 | 2 | 3 |
| 22 | 0 | 0 | 0 | 0 | 0 | - | 0 | 0 | 0 | 1 | 1 |
| 23 | 3 | 0 | 3 | 3 | 3 | - | 0 | 2 | 0 | 3 | 3 |
| 24 | 3 | 0 | 1 | 3 | 3 | - | 0 | 0 | 0 | 3 | 3 |
| 28 | 0 | 1 | 0 | 1 | 2 | 0 | 0 | 0 | 0 | 1 | 0 |
| 29 | 0 | 0 | 0 | 0 | 3 | 0 | 0 | 0 | 0 | 0 | 0 |
| 30 | 0 | 0 | 0 | 0 | 0 | 0 | 0 | 0 | 0 | 3 | 3 |
| 31 | 0 | 0 | 1 | 1 | 0 | 0 | 0 | 0 | - | 1 | 3 |
| 32 | 3 | 1 | 3 | 3 | 3 | 3 | 3 | 3 | 3 | 3 | 3 |
| 33 | 1 | 0 | 0 | 2 | 3 | 3 | 0 | 0 | 3 | 3 | 3 |
| 34 | 3 | 3 | 3 | 3 | 3 | 3 | 3 | 3 | 3 | 3 | 3 |
| 35 | 3 | 3 | 3 | 3 | 3 | 3 | 3 | 3 | 3 | 3 | 3 |
| 36 | 3 | 3 | 3 | 3 | 3 | 3 | 3 | 3 | 3 | 3 | 3 |
| 37 | 3 | 1 | 3 | 3 | 3 | 3 | 3 | 3 | 3 | 3 | 3 |
| 40 | 0 | N | 0 | 0 | 1 | 1 | 0 | 2 | N | 3 | 3 |
| 41 | 3 | 1 | 3 | 3 | 3 | 3 | 0 | 3 | - | 3 | 3 |
| 42 | 0 | 0 | 0 | 3 | 0 | 0 | 0 | 0 | 0 | 2 | 3 |
| 43 | 3 | 3 | 3 | 3 | 3 | 3 | 3 | 3 | 3 | 3 | 3 |
| 44 | 3 | 0 | 3 | 3 | 3 | 3 | 1 | 3 | 3 | 3 | 3 |
| 45 | 3 | 1 | 3 | 3 | 3 | 3 | 1 | 3 | 3 | 3 | 3 |
| 46 | 3 | 3 | 3 | 3 | 3 | 3 | 0 | 1 | 2 | 3 | 3 |

The compounds were further tested by utilizing the above procedure on the following plant species:

| | |
|---|---|
| L-Soybean | R-Hemp Sesbania |
| M-Sugarbeet | E-Lambsquarters |
| N-Wheat | F-Smartweed |
| O-Rice | C-Velvetleaf |
| P-Sorghum | J-Downy Brome |
| B-Cocklebur | S-Panicum |
| Q-Wild Buckwheat | K-Barnyardgrass |
| D-Morningglory | T-Crabgrass |

The results are summarized in Table V.

TABLE V

Pre-Emergent

| Compound of Example No. | Kg/ha | L | M | N | O | P | B | Q | D | R | E | F | C | J | S | K | T |
|---|---|---|---|---|---|---|---|---|---|---|---|---|---|---|---|---|---|
| | | Plant Species | | | | | | | | | | | | | | | |
| 4 | 5.6 | 0 | 1 | 0 | 0 | 2 | 0 | 0 | 0 | 0 | 3 | 1 | 0 | 3 | 2 | 3 | 3 |
| 6 | 5.6 | 3 | 3 | 3 | 3 | 3 | 1 | 2 | 2 | 2 | 3 | 3 | 2 | 3 | 3 | 3 | 3 |
| | 1.12 | 1 | 2 | 3 | 2 | 0 | 0 | 0 | 0 | 1 | 3 | 1 | 1 | 3 | 3 | 3 | 3 |
| | .27 | 0 | 1 | 0 | 0 | 0 | 0 | 0 | 0 | 0 | 2 | 0 | 0 | 1 | 1 | 1 | 2 |
| 7 | 5.6 | 3 | 3 | 3 | 3 | 3 | 0 | 3 | 3 | 2 | 3 | 1 | 1 | 3 | 3 | 3 | 3 |
| | 1.12 | 0 | 1 | 1 | 0 | 0 | 0 | 0 | 1 | 1 | 0 | 0 | 0 | 0 | 1 | 3 | 3 |
| | .27 | 1 | 1 | 0 | 0 | 0 | 0 | 1 | 0 | 0 | 0 | 0 | 0 | 0 | 0 | 1 | 1 |
| 16a | 5.6 | 3 | 3 | 3 | 3 | 3 | 0 | 2 | 3 | 3 | 3 | 3 | 2 | 3 | 3 | 3 | 3 |
| | 1.12 | 0 | 2 | 3 | 0 | 2 | 0 | 1 | 1 | 2 | 1 | 0 | 0 | 2 | 3 | 3 | 3 |
| 12 | 5.6 | 1 | 3 | 3 | 3 | 3 | 1 | 3 | 2 | 3 | 3 | 3 | 1 | 3 | 3 | 3 | 3 |
| | 1.12 | 0 | 1 | 0 | 0 | 0 | 3 | 0 | 0 | 0 | 0 | 0 | 0 | 0 | 0 | 3 | 3 |
| 9 | 5.6 | 0 | 1 | 1 | 0 | 0 | 0 | 1 | 0 | 0 | 0 | 1 | 0 | 0 | 1 | 3 | - |
| 13 | 5.6 | 0 | 2 | 1 | 1 | 1 | 0 | 3 | 0 | 2 | 3 | 3 | 0 | 3 | 1 | 3 | 3 |
| | 1.12 | 0 | 1 | 0 | 0 | 0 | 0 | 1 | 0 | 0 | 0 | 0 | 0 | 0 | 0 | 1 | 0 |
| | .27 | 0 | 1 | 0 | 0 | 0 | 0 | 1 | 0 | 0 | 1 | 1 | 0 | 0 | 0 | 0 | 0 |
| 10 | 11.2 | 3 | 3 | 3 | 3 | 3 | 3 | 2 | 3 | 3 | 2 | 2 | 1 | 3 | 3 | 3 | - |
| | 5.6 | 1 | 3 | 3 | 3 | 3 | 0 | 1 | 3 | 3 | 2 | 2 | 1 | 3 | 3 | 3 | - |
| | 1.12 | 1 | 1 | 0 | 2 | 0 | 0 | 1 | 1 | 3 | 2 | 1 | 0 | 0 | 0 | 2 | - |
| | .27 | 0 | 1 | 0 | 0 | 0 | 3 | 1 | 0 | 3 | 0 | 3 | 0 | 0 | 0 | 0 | - |
| 3 | 5.6 | 1 | 0 | 0 | 0 | 0 | 0 | 0 | 0 | 1 | 0 | 1 | 0 | 0 | 0 | 3 | - |
| | 1.12 | 2 | 0 | 1 | 0 | 0 | 0 | 0 | 0 | 3 | 0 | 0 | 0 | 0 | 0 | 0 | - |
| 14 | 5.6 | 2 | 0 | 2 | 2 | 3 | 0 | 2 | 3 | 2 | 3 | 3 | 1 | 3 | 3 | 3 | 3 |
| 18 | 5.6 | 3 | 3 | 3 | 3 | 3 | 0 | 3 | 3 | 3 | 3 | 3 | 1 | 3 | 3 | 3 | 3 |
| | 1.12 | 0 | 0 | 0 | 2 | 0 | 0 | 0 | 0 | 0 | 0 | 0 | 0 | 0 | 2 | 2 | 3 |
| 23 | 5.6 | 1 | 3 | 1 | 3 | 3 | 1 | 3 | 2 | 3 | 3 | - | 2 | 3 | 3 | 3 | 3 |
| | 1.12 | 1 | 1 | 0 | 3 | 3 | 0 | 1 | 0 | 1 | 2 | - | 0 | 2 | 3 | 3 | 3 |
| | .27 | 0 | 2 | 0 | 1 | 1 | 0 | 0 | 1 | 1 | 2 | - | 2 | 1 | 1 | 2 | 3 |
| 24 | 5.6 | 0 | 2 | 1 | 2 | 3 | 0 | 3 | 1 | 2 | 3 | - | 2 | 3 | 3 | 3 | 3 |
| | 1.12 | 0 | 1 | 0 | 2 | 0 | 0 | 0 | 0 | 0 | 2 | - | 1 | 0 | 1 | 3 | 3 |
| 2 | 5.6 | 3 | 3 | 3 | 3 | 3 | 0 | 3 | 3 | 3 | 3 | - | 3 | 3 | 3 | 3 | 3 |
| | 1.12 | 2 | 2 | 3 | 3 | 3 | 0 | 3 | 2 | 2 | 3 | - | 1 | 3 | 3 | 3 | 3 |
| | .27 | 0 | 1 | 1 | 1 | 3 | 0 | 3 | 1 | 1 | 1 | - | 0 | 3 | 3 | 3 | 3 |
| | .056 | 0 | 2 | 0 | 0 | 1 | 0 | 2 | 0 | 0 | 1 | - | 0 | 2 | 2 | 3 | 2 |
| | .011 | 0 | 2 | 1 | 2 | 1 | 0 | 0 | 0 | 0 | 2 | - | 0 | 1 | 1 | 2 | 2 |
| 21 | 5.6 | 0 | 0 | 0 | 0 | 0 | 0 | 0 | 0 | 0 | 0 | - | 0 | 2 | 1 | 3 | 3 |
| 20 | 5.6 | 0 | 3 | 0 | 0 | 0 | 0 | 0 | 1 | 0 | 0 | - | 0 | 0 | 0 | 1 | 2 |
| 30 | 5.6 | 0 | 0 | 0 | 0 | 0 | 0 | 0 | 0 | 0 | 1 | 1 | 0 | 0 | 0 | 2 | 2 |

The herbicidal compositions of this invention, including concentrates which require dilution prior to application, may contain at least one active ingredient and an adjuvant in liquid or solid form. The compositions can be prepared by admixing the active ingredient with an adjuvant including diluents, extenders, carriers, and conditioning agents to provide compositions in the form of finely-divided particulate solids, granules, pellets, solutions, dispersions, or emulsions. Thus the active ingredient can be used with an adjuvant such as a finely-divided solid, a liquid of organic origin, water, a wetting agent, a dispersing agent, an emulsifying agent, or any suitable combination of these.

The compositions of this invention, particularly liquids and wettable powders, may contain as a conditioning agent one or more surface-active agents in amounts sufficient to render a given composition readily dispersible in water or in oil. The incorporation of a surface-active agent into such compositions normally greatly enhances their efficacy. By the term "surface-active agent" it is understood that wetting agents, dispersing agents, suspending agents, and emulsifying agents are included therein. Anionic, cationic, and non-ionic agents can be used with equal facility.

Typical wetting agents are alkyl benzene and alkyl naphthalene sulfonates, sulfated fatty alcohols, amines or acid amides, long chain acid esters of sodium isothionate, esters of sodium sulfosuccinate, sulfated or sulfonated fatty acid esters, petroleum sulfonates, sulfonated vegetable oils, ditertiary acetylenic gylcols, polyoxyethylene derivatives of alkylphenols (particularly isooctylphenol and nonylphenol) and polyoxyethylene derivatives of the mono-higher fatty acid esters of hexitol anhydrides (e.g., sorbitan). Preferred dispersants are methyl cellulose, polyvinyl alcohol, sodium lignin sulfonates, polymeric alkyl, naphthalene sulfonates, sodium naphthalene sulfonate, and the polymethylene bisnaphthalene sulfonate.

Wettable powders are water-dispersible compositions containing one or more active ingredients, an inert solid extender, and one or more wetting and dispersing agents. The inert solid extenders are usually of mineral origin such as the natural clays, diatomaceous earth and synthetic minerals derived from silica and the like. Examples of such extenders include kaolinites, attapulgite clay and synthetic magnesium silicate. The wettable powder compositions of this invention usually contain from 0.5 to 60 parts (preferably from 5—20 parts) of active ingredient, from 0.25 to 25 parts (preferably 1—15 parts) of wetting agent, from about 0.25 to 25 parts (preferably 1.0—15 parts) of dispersant and from 5 to about 95 parts (preferably 5—50 parts) of inert solid extender, all parts being by weight of the total composition. Where required, from about 0.1 to 2.0 parts of the solid inert extender can be replaced by a corrosion inhibitor or anti-foaming agent or both.

Other formulations include dust concentrates comprising from 0.1 to 60% by weight of the active ingredient on a suitable extender; these dusts may be diluted for application at concentrations within the range of from about 0.1—10% by weight.

Aqueous suspensions or emulsions may be prepared by stirring an aqueous mixture of a water-insoluble active ingredient and an emulsification agent until uniform and then homogenized to give a stable emulsion of very finely-divided particles. The resulting concentrated aqueous suspension is characterized by its extremely small particle size, so that when diluted and sprayed, coverage is very uniform. Suitable concentrations of these formulations contain from about 0.1—60%, preferably 5—50%, by weight of active ingredient, the upper limit being determined by the solubility limit of active ingredient in the solvent.

In another form of aqueous suspension, a water-immiscible herbicide is encapsulated to form a microencapsulated phase dispersed in an aqueous phase. In one embodiment, minute capsules are formed by bringing together an aqueous phase containing a lignin sulfonate emulsifier and a water-immiscible chemical and polymethylene polyphenylisocyanate, dispersing the water-immiscible phase in the aqueous phase followed by addition of a polyfunctional amine. The isocyanate and amine compounds react to form a solid urea shell wall around particles of the water-immiscible chemical, thus forming microcapsules thereof. Typically, the concentration of the microencapsulated material will range from 480 to 700 g/l of total composition, preferably 480 to 600 g/l.

Concentrates are usually solutions of active ingredient in water-immiscible or partially water-immiscible solvents together with a surface active agent. Suitable solvents for the active ingredient of this invention include dimethylformamide, dimethylsulfoxide, N-methylpyrrolidone, hydrocarbons, and water-immiscible ethers, esters or ketones. However, other high strength liquid concentrates may be formulated by dissolving the active ingredient in a solvent then diluting, e.g., with kerosene, to spray concentration.

The concentrate compositions herein generally contain from about 0.1 to 95 parts (preferably 5—60 parts) active ingredient, about 0.25 to 50 parts (preferably 1—25 parts) surface active agent and where required, about 4 to 94 parts solvent, all parts being by weight based on the total weight of emulsifiable oil.

Granules are physically stable particulate compositions comprising active ingredient adhering to or distributed through a basic matrix of an inert, finely-divided particulate extender. In order to aid leaching of the active ingredient from the particulate, a surface active agent such as those listed hereinbefore can be present in the composition. Natural clays, pyrophyllites, illite, and vermiculite are examples of operable classes of particulate mineral extenders. The preferred extenders are the porous, absorptive, preformed particles such as preformed and screened particulate attapulgite or heat expanded, particulate vermiculite and the finely-divided clays such as kaolin clays, hydrated attapulgite or bentonitic clays. These extenders are sprayed or blended with the active ingredient to form the herbicidal granules.

The granular compositions of this invention may contain from about 0.1 to about 30 parts by weight of active ingredient per 100 parts by weight of clay and 0 to about 5 parts by weight of surface active agent per 100 parts by weight of particulate clays.

The compositions of this invention can also contain other additaments, for example, fertilizers, other herbicides, other pesticides, safeners, and the like used as adjuvants or in combination with any of the above-described adjuvants. Chemicals useful in combination with the active ingredients of this invention, for example, triazines, ureas, carbamates, acetamides, acetanilides, uracils, acetic acid or phenol derivatives, thiolcarbamates, triazoles, benzoic acids, nitriles, biphenyl ethers and the like such as:

Heterocyclic Nitrogen/Sulfur Derivatives

2-Chloro-4-ethylamino-6-isopropylamino-*s*-triazine
2-Chloro-4,6-bis-(isopropylamino)-*s*-triazine
2-Chloro-4,6-bis-(ethylamino)-*s*-triazine
3-Isopropyl-1H-2,1,3-benzothiadiazin-4-(3H)-one 2,2 dioxide
3-Amino-1,2,4-triazole
6,7-Dihydrodipyrido(1,2-a:2′,1′-c)-pyrazidinium salt
5-Bromo-3-isopropyl-6-methyluracil
1,1′-Dimethyl-4,4′-bipyridinium

Ureas

N′-(4-chlorophenoxy)phenyl-N,N-dimethylurea
N,N-dimethyl-N′-(3-chloro-4-methylphenyl) urea
3-(3,4-dichlorophenyl)-1,1-dimethylurea
1,3-Dimethyl-3-(2-benzothiazolyl) urea
3-(*p*-Chlorophenyl)-1,1-dimethylurea
1-Butyl-3-(3,4-dichlorophenyl)-1-methylurea

Carbamates/Thiolcarbamates

2-Chloroallyl diethyldithiocarbamate
S-(4-chlorobenzyl)-N,N-diethylthiocarbamate
Isopropyl N-(3-chlorophenyl) carbamate
*S*-2,3-dichloroallyl-N,N-diisopropylthiolcarbamate
Ethyl N,N-dipropylthiolcarbamate
*S*-propyl dipropylthiolcarbamate

Acetamides/Acetanilides/Anilines/Amides

2-Chloro-N,N-diallylacetamide
N,N-dimethyl-2,2-diphenylacetamide
N-(2,4-dimethyl-5-[[(trifluoromethyl)sufonyl]amino]phenyl)acetamide
N-Isopropyl-2-chloroacetanilide
2′,6′-Diethyl-N-methoxymethyl-2-chloroacetanilide
2′-Methyl-6′-ethyl-N-(2-methoxyprop-2-yl)-2-chloroacetanilide
α,α,α-Trifluoro-2,6-dinitro-*N*,N-dipropyl-*p*-toluidine
N-(1,1-dimethylpropynyl)-3,5-dichlorobenzamide

Acids/Esters/Alcohols

2,2-Dichloropropionic acid
2-Methyl-4-chlorophenoxyacetic acid
2,4-Dichlorophenoxyacetic acid
Methyl-2-[4-(2,4-dichlorophenoxy)phenoxy]propionate
3-Amino-2,5-dichlorobenzoic acid
2-Methoxy-3,6-dichlorobenzoic acid
2,3,6-Trichlorophenylacetic acid
*N*-1-naphthylphthalamic acid
Sodium 5-[2-chloro-4-(trifluoromethyl)phenoxy]-2-nitrobenzoate
4,6-Dinitro-*o*-*sec*-butylphenol
N-(phosphonomethyl) glycine and its $C_{1-6}$ monoalkyl amine and alkaline metal salts and combinations thereof

Ethers

2,4-Dichlorophenyl-4-nitrophenyl ether
2-Chloro-α,α,α-trifluoro-*p*-tolyl-3-ethoxy-4-nitrodiphenyl ether

Miscellaneous

2,6-Dichlorobenzonitrile
Monosodium acid methanearsonate
Disodium methanearsonate

Fertilizers useful in combination with the active ingredients include, for example, ammonium nitrate, urea, potash, and superphosphate. Other useful additaments include materials in which plant organisms take root and grow such as compost, manure, humus, sand and the like.

Herbidical formulations of the types described above are exemplified in several illustrative embodiments below.

I. Emulsifiable Concentrates

| | | Weight Percent |
|---|---|---|
| A. | Compound of Example No. 5 | 1.0 |
| | Free acid of complex organic phosphate of aromatic or aliphatic hydrophobe base (e.g., GAFAC RE-610, registered trademark of GAF Corp.) | 5.59 |
| | Polyoxyethylene/polyoxyproylene block copolymer with butanol (e.g., Tergitol XH, registered trademark of Union Carbide Corp.) | 1.11 |
| | Phenol | 5.34 |
| | Monochlorobenzene | 86.96 |
| | | 100.00 |
| B. | Compound of Example No. 16 | 25.00 |
| | Free acid of complex organic phosphate or aromatic or aliphatic hydrophobe base (e.g. GAFAC RE-610) | 5.00 |
| | Polyoxyethylene/polyoxypropylene block copolymer with butanol (e.g. Tergitol XH) | 1.60 |
| | Phenol | 4.75 |
| | Monochlorobenzene | 63.65 |
| | | 100.00 |

II. Flowables

| | | Weight Percent |
|---|---|---|
| A. | Compound of Example No. 6 | 25.00 |
| | Methyl cellulose | 0.3 |
| | Silica aerogel | 1.5 |
| | Sodium lignosulfonate | 3.5 |
| | Sodium N-methyl-n-oleyl taurate | 2.0 |
| | Water | 67.7 |
| | | 100.0 |

| | | |
|---|---|---|
| B. | Compound of Example No. 17 | 45.0 |
| | Methyl cellulose | .3 |
| | Silica aerogel | 1.5 |
| | Sodium lignosulfonate | 3.5 |
| | Sodium N-methyl-N-oleyl taurate | 2.0 |
| | Water | 47.3 |
| | | 100.0 |

III. Wettable Powders

| | | Weight Percent |
|---|---|---|
| A. | Compound of Example No. 5 | 25.0 |
| | Sodium lignosulfonate | 3.0 |
| | Sodium N-methyl-N-oleyl-taurate | 1.0 |
| | Amorphous silica (synthetic) | 71.0 |
| | | 100.0 |
| B. | Compound of Example No. 3 | 80.0 |
| | Sodium dioctyl sulfosuccinate | 1.25 |
| | Calcium lignosulfonate | 2.75 |
| | Amorphous silica (synthetic) | 16.00 |
| | | 100.00 |
| C. | Compound of Example No. 6 | 10.0 |
| | Sodium lignosulfonate | 3.0 |
| | Sodium N-methyl-N-oleyl-taurate | 1.0 |
| | Kaolinite clay | 86.0 |
| | | 100.0 |

VI. Water-Soluble Powders

| | | Weight Percent |
|---|---|---|
| A. | Compound of Example No. 1 | 10.0 |
| | Sodium dioctyl sulfosuccinate | 2.0 |
| | Silica aerogel | 5.0 |
| | Methyl violet | 0.1 |
| | Sodium carbonate | 82.9 |
| | | 100.0 |

| | |
|---|---|
| B. Compound of Example No. 17 | 90.0 |
| Ammonium phosphate | 10.0 |
| | 100.0 |

V. Dusts

| | Weight Percent |
|---|---|
| A. Compound of Example No. 2 | 2.0 |
| Attapulgite | 98.0 |
| | 100.0 |
| B. Compound of Example No. 9 | 60.0 |
| Montmorillonite | 40.0 |
| | 100.0 |
| C. Compound of Example No. 7 | 30.0 |
| Ethylene glycol | 1.0 |
| Bentonite | 69.0 |
| | 100.0 |
| D. Compound of Example No. 16 | 1.0 |
| Diatomaceous earth | 99.0 |
| | 100.0 |

VI. Granules

| | Weight Percent |
|---|---|
| A. Compound of Example No. 8 | 15.0 |
| Granular attapulgite (20/40 mesh) | 85.0 |
| | 100.0 |
| B. Compound of Example No. 9 | 30.0 |
| Diatomaceous earth (20/40) | 70.0 |
| | 100.0 |
| C. Compound of Example No. 6 | 1.0 |
| Ethylene glycol | 5.0 |
| Methylene blue | 0.1 |
| Pyrophyllite | 93.9 |
| | 100.0 |

| | |
|---|---|
| D. Compound of Example No. 13 | 5.0 |
| Pyrohyllite (20/40) | 95.0 |
| | 100.0 |

When operating in accordance with the present invention, effective amounts of the compounds of this invention are applied to the soil containing the plants, in any convenient fashion. The application of liquid and particulate solid compositions to the soil can be carried out by conventional methods, e.g., power dusters, boom and hand sprayers, and spray dusters. The compositions can also be applied from airplanes as a dust or a spray because of their effectiveness at low dosages.

The application of an effective amount of the compounds of this invention to the locus of undesired weeds is essential and critical for the practice of the present invention. The exact amount of active ingredient to be employed is dependent upon various factors, including the plant species and stage of development thereof, the type and condition of soil, the amount of rainfall and the specific compounds employed. In selective preemergence application to the soil a dosage of from 0.1 to about 11.2 kg/ha, preferably from about 1.12 to about 5.60 kg/ha is usually employed. Lower or higher rates may be required in some instances. One skilled in the art can readily determine from this specification, including the above examples, the optimum rate to be applied in any particular case.

The term "soil" is employed in its broadest sense to be inclusive of all conventional "soils" as defined in Webster's New International Dictionary, Second Edition, Unabridged (1961). Thus the term refers to any substance or medium in which vegetation may take root and grow, and includes not only earth, but also compost, manure, muck, humus, sand and the like, adapted to support plant growth.

Although the invention is described with respect to specific modifications, the details thereof are not to be construed as limitations except to the extent indicated in the following claims.

## Claims

1. Herbicidal compositions comprising an adjuvant and a herbicidally effective amount of a compound of the formula selected from

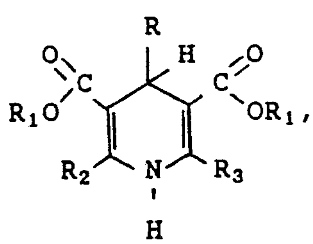,

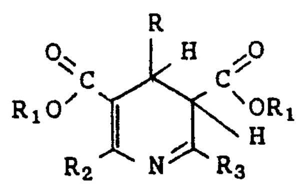

and

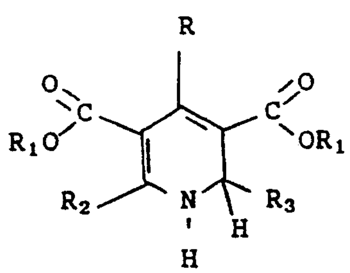

wherein R is selected from phenyl, $C_{1-6}$ alkyl, $C_{1-6}$ haloalkyl, $C_{1-6}$ alkoxy $C_{1-6}$ alkyl, $C_{1-6}$ alkylthio $C_{1-6}$ alkyl, $C_{1-6}$ alkylcarbonylalkyl $C_{1-6}$ alkyl, $C_3$—$C_6$ cycloalkyl, $C_{3-6}$ cycloalkyl $C_{1-6}$ alkyl, phenylmethoxymethyl, phenoxy $C_{1-6}$ alkyl, pyridyl, furyl and thienyl radicals; each $R_1$ is independently selected from $C_{1-4}$ alkyl radicals and $R_2$ and $R_3$ are independently selected from $C_{1-6}$ alkyl and fluorinated methyl radicals, provided that at least one of $R_2$ and $R_3$ must be fluorinated methyl.

2. Compositions of Claim 1 wherein R is $C_{1-6}$ alkyl.

3. Compositions of Claim 2 wherein one of $R_2$ and $R_3$ is a trifluoromethyl radical.

4. Compositions of Claim 2 wherein one of $R_2$ and $R_3$ is a difluoromethyl radical.

5. Compositions of Claim 1 wherein R is selected from alkyl $C_{2-4}$ and cyclopropylmethyl.

6. Compositions of Claim 5 wherein $R_1$ is alkyl $C_{2-4}$.

7. Compositions of Claim 6 wherein one of $R_2$ and $R_3$ is a trifluoromethyl radical.

8. Compositions of Claim 6 wherein one of $R_2$ and $R_3$ is a difluoromethyl radical.

9. Compositions of Claim 6 wherein $R_2$ is fluoroalkyl and $R_3$ is alkyl.

10. Compositions of Claim 1 wherein R is isobutyl.

11. Compositions of Claim 10 wherein $R_1$ is methyl.

12. Compositions of Claim 11 wherein one of $R_2$ and $R_3$ is a trifluoromethyl radical.

13. Compositions of Claim 11 wherein one of $R_2$ and $R_3$ is difluoromethyl radical.

14. Compositions of Claim 1 wherein R is furyl.

15. Compositions of Claim 1 wherein R is pyridyl.

16. Compositions of Claim 1 wherein R is thienyl.

17. Compositions of Claim 1 wherein R is methylthiomethyl.

18. Compositions of Claim 1 wherein R is methoxymethyl.

19. Compositions of Claim 1 wherein R is methylthioethyl.

20. Compositions of Claim 1 wherein R is ethoxyethyl.

21. Compositions of Claim 1 wherein R is ethyl.

22. A method for controlling undesirable plants which comprises applying to the locus thereof a herbicidally effective amount of a compound of the formula selected from:

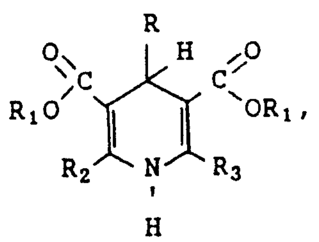,

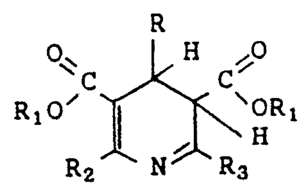

and

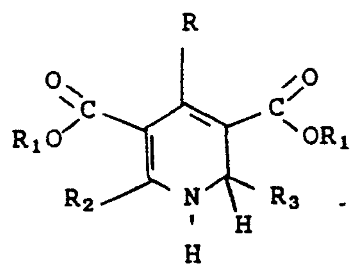.

wherein R is selected from phenyl, $C_{1-6}$ alkyl, $C_{1-6}$ haloalkyl, $C_{1-6}$ alkoxy $C_{1-6}$ alkyl, $C_{1-6}$ alkylthio $C_{1-6}$ alkyl, $C_{1-6}$ alkylcarbonyloxy $C_{1-6}$ alkyl, $C_3$—$C_6$ cycloalkyl, $C_{3-6}$ cycloalkyl $C_{1-6}$ alkyl, phenylmethoxymethyl, phenoxy $C_{1-6}$ alkyl, pyridyl, furyl and thienyl radicals; each $R_1$ is independently selected from $C_{1-4}$ alkyl radicals and $R_2$ and $R_3$ are independently selected from $C_{1-6}$ alkyl and fluorinated methyl radicals, provided that at least one of $R_2$ and $R_3$ must be fluorinated methyl.

23. The method of Claim 22 wherein each of $R_2$ and $R_3$ is independently selected from fluorinated methyl radicals.

24. The method of Claim 23 wherein R is $C_{1-6}$ alkyl.

25. The method of Claim 24 wherein one of $R_2$ and $R_3$ is a trifluoromethyl radical.

26. The method of Claim 24 wherein one of $R_2$ and $R_3$ is a difluoromethyl radical.

27. The method of Claim 22 wherein R is selected from alkyl $C_{2-4}$ and cyclopropylmethyl.

28. The method of Claim 27 wherein $R_1$ is alkyl $C_{2-4}$.

29. The method of Claim 28 wherein one of $R_2$ and $R_3$ is a trifluoromethyl radical.

30. The method of Claim 28 wherein one of $R_2$ and $R_3$ is a difluoromethyl radical.

31. The method of Claim 28 wherein $R_2$ is fluorinated methyl and $R_3$ is alkyl.

32. The method of Claim 22 wherein R is isobutyl.

33. The method of Claim 32 wherein $R_1$ is methyl.

34. The method of Claim 33 wherein one of $R_2$ and $R_3$ is a trifluoromethyl radical.

35. The method of Claim 33 wherein one of $R_2$ and $R_3$ is a difluoromethyl radical.

36. The method of Claim 22 wherein R is furyl.

37. The method of Claim 22 wherein R is pyridyl.

38. The method of Claim 22 wherein R is thienyl.

39. The method of Claim 23 wherein R is methylthiomethyl.

40. The method of Claim 23 wherein R is methoxymethyl.

41. The method of Claim 23 wherein R is methylthioethyl.

42. The method of Claim 23 wherein R is ethoxyethyl.

43. The method of Claim 23 wherein R is ethyl.

44. Compounds having the formula selected from:

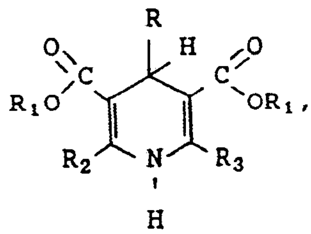,
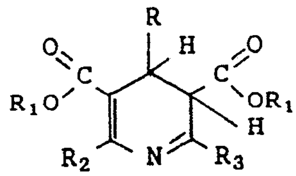 and

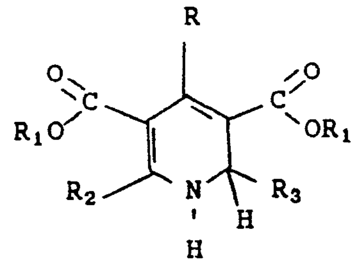

wherein R is selected from phenyl, $C_{1-6}$ alkyl, $C_{1-6}$ haloalkyl, $C_{1-6}$ hydroxyalkyl, $C_{1-6}$ alkoxy $C_{1-6}$ alkyl, $C_{1-6}$ alkylthio $C_{1-6}$ alkyl, $C_{1-6}$ alkylcarbonyloxy $C_{1-6}$ alkyl, $C_3$—$C_6$ cycloalkyl, $C_3$—$C_6$ cycloalkyl $C_{1-6}$ alkyl, phenylmethoxymethyl, phenoxy $C_{1-6}$ alkyl, pyridyl furyl and thienyl radicals; each $R_1$ is independently selected from $C_{1-4}$ alkyl radicals and $R_2$ and $R_3$ are independently selected from $C_{1-6}$ alkyl and fluorinated methyl radicals, provided that at least one of $R_2$ and $R_3$ must be flourinated methyl.

45. Compounds according to Claim 44 wherein R is alkyl $C_{1-6}$ and $R_1$ is ethyl.

46. Compounds according to Claim 44 wherein R is alkyl $C_{1-6}$ and $R_1$ is methyl.

47. Compounds according to Claim 44 wherein one of $R_2$ and $R_3$ is a trifluoromethyl radical.

48. Compounds according to Claim 44 wherein one of $R_2$ and $R_3$ is a difluoromethyl radical.

49. Compounds according to Claim 44 wherein R is selected from alkyl $C_{2-4}$ and cyclopropylmethyl.

50. Compounds according to Claim 49 wherein one of $R_2$ and $R_3$ is trifluoromethyl radical.

51. Compounds according to Claim 49 wherein one of $R_2$ and $R_3$ is a difluoromethyl radical.

52. Compounds according to Claim 49 wherein $R_2$ is fluorinated methyl and $R_3$ is alkyl.

53. Compounds according to Claim 44 wherein R is isobutyl.

54. Compounds according to Claim 49 wherein $R_1$ is selected from methyl and ethyl.

55. Compounds according to Claim 54 wherein R is furyl.

56. Compounds according to Claim 44 wherein R is pyridyl.

57. Compounds according to Claim 44 wherein R is thienyl.

58. Compounds according to Claim 44 wherein R is an $C_1$—$C_6$ alkythio $C_1$—$C_6$ alkyl radical.

59. Compounds according to Claim 58 wherein R is a methylthiomethyl radical.

60. Compounds according to Claim 58 wherein R is a methylthioethyl radical.

61. Compounds according to Claim 44 wherein R is halo $C_{1-6}$ alkyl.

62. Compounds according to Claim 61 wherein R is selected from chloromethyl, trifluoromethyl and 2-(trifluoromethyl)propyl.

63. Compounds according to Claim 44 wherein R is a $C_{1-6}$ alkylcarbonyloxy $C_{1-6}$ alkyl.

64. Compounds according to Claim 63 wherein R is acetoxymethyl.

65. Compounds according to Claim 44 wherein R is a $C_{1-6}$ alkoxy-$C_{1-6}$ alkyl radical.

66. Compounds according to Claim 65 wherein R is methoxymethyl.

67. Compounds according to Claim 65 wherein R is ethoxyethyl.

68. The process for preparing dihydropyridines having a formula selected from

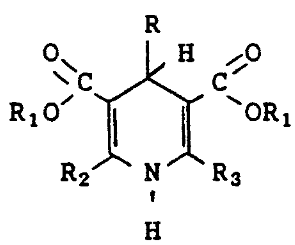 and 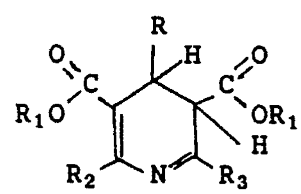

wherein R is selected from phenyl, $C_{1-6}$ alkyl, $C_{1-6}$ haloalkyl, $C_{1-6}$ hydroxyalkyl, $C_{1-6}$ alkoxy $C_{1-6}$ alkyl, $C_{1-6}$ alkylthio $C_{1-6}$ alkyl, $C_{1-6}$ alkylcarbonyloxy $C_{1-6}$ alkyl, $C_3$—$C_6$ cycloalkyl $C_{3-6}$ cycyloalkyl $C_{1-6}$ alkyl, phenylmethoxymethyl, phenoxy $C_{1-6}$ alkyl, pyridyl, furyl and thienyl radicals; each $R_1$ is independently selected from $C_{1-4}$ alkyl radicals and $R_2$ and $R_3$ are the same fluorinated methyl radicals in a single reaction mixture which comprises the steps of:

(1) reacting an alkyl 3-ketoester represented by the formula

$$R_2\overset{O}{\overset{\|}{C}}CH_2\overset{O}{\overset{\|}{C}}OR_1 \quad \text{and} \quad R_3\overset{O}{\overset{\|}{C}}\text{-}CH_2\overset{O}{\overset{\|}{C}}\text{-}OR_1$$

wherein $R_1$, $R_2$, and $R_3$ are as defined above, with an aldehyde represented by the formula

$$R\overset{O}{\overset{\|}{C}}H$$

wherein R is as defined above;

(2) adding a reactant selected from ammonium hydroxide and gaseous ammonia through the reaction product of step (1) in the presence of an aprotic inert solvent to provide a dihydropiperidine and

(3) dehydrating said dihydroxypiperidine with dehydrating agent to provide the said dihydropyridine.

69. The process of Claim 68 wherein the alkyl 3-ketoester is ethyl trifluoroacetoacetate.

70. The process of Claim 68 wherein the alkyl 3-ketoester is ethyl difluoroacetoacetate and methyl trifluoroacetoacetate.

71. The process of Claim 68 wherein the aldehyde is propionaldehyde.

72. The process of Claim 68 wherein the aldehyde is butyraldehyde.

73. The process of Claim 68 wherein the aldehyde is isovaleraldehyde.

**Patentansprüche**

1. Herbizide Zusammensetzungen umfassend ein Adjuvans und einen herbizid wirksamen Anteil einer Verbindung der Formel ausgewählt aus

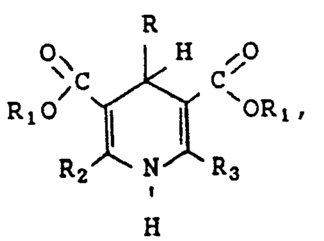

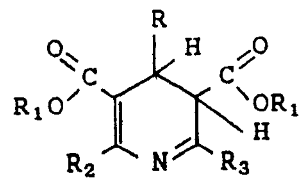

und

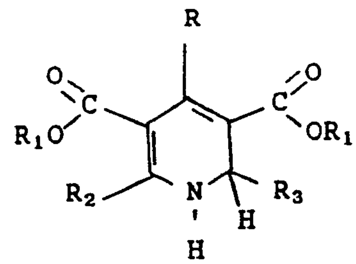

worin R ausgewählt ist aus Phenyl-, $C_{1-6}$-Alkyl-, $C_{1-6}$-Halogenalkyl-, $C_{1-6}$-Alkoxy-$C_{1-6}$-alkyl, $C_{1-6}$-Alkylthio-$C_{1-6}$-alkyl-, $C_{1-6}$-Alkylcarbonyloxy-$C_{1-6}$-alkyl, $C_{3-6}$-Cycloalkyl-, $C_{3-6}$-Cycloalkyl-$C_{1-6}$-alkyl, Phenylmethoxymethyl, Phenoxy-$C_{1-6}$-alkyl, Pyridyl, Furyl- und Thienylresten; jedes $R_1$ unabhängig ausgewählt ist aus $C_{1-4}$-Alkylresten und $R_2$ und $R_3$ unabhängig ausgewählt sind aus $C_{1-6}$-Alkyl- und fluorierten Methylresten, vorausgesetzt, daß mindestens eines von $R_2$ und $R_3$ fluoriertes Methyl sein muß.

2. Zusammensetzungen nach Anspruch 1, worin R $C_{1-6}$-Alkyl ist.

3. Zusammensetzungen nach Anspruch 2, worin eines von $R_2$ und $R_3$ ein Trifluormethylrest ist.

4. Zusammensetzungen nach Anspruch 2, worin eines von $R_2$ und $R_3$ ein Difluormethylrest ist.

5. Zusammensetzungen nach Anspruch 1, worin R ausgewählt ist aus $C_{2-4}$-Alkyl und Cyclopropylmethyl.

6. Zusammensetzungen nach Anspruch 5, worin $R_1$ $C_{2-4}$-Alkyl ist.

7. Zusammensetzungen nach Anspruch 6, worin eines von $R_2$ und $R_3$ ein Trifluormethylrest ist.

8. Zusammensetzungen nach Anspruch 6, worin eines von $R_2$ und $R_3$ ein Difluormethylrest ist.

9. Zusammensetzungen nach Anspruch 6, worin $R_2$ Fluroalkyl und $R_3$ Alkyl sind.

10. Zusammensetzungen nach Anspruch 1, worin R Isobutyl ist.

11. Zusammensetzungen nach Anspruch 10, worin $R_1$ Methyl ist.

12. Zusammensetzungen nach Anspruch 11, worin eines von $R_2$ und $R_3$ ein Trifluormethylrest ist.

13. Zusammensetzungen nach Anspruch 11, worin eines von $R_2$ und $R_3$ ein Difluormethylrest ist.

14. Zusammensetzungen nach Anspruch 1, worin R Furyl ist.

15. Zusammensetzungen nach Anspruch 1, worin R Pyridyl ist.

16. Zusammensetzungen nach Anspruch 1, worin R Thienyl ist.

17. Zusammensetzungen nach Anspruch 1, worin R Methylthiomethyl ist.
18. Zusammensetzungen nach Anspruch 1, worin R Methoxymethyl ist.
19. Zusammensetzungen nach Anspruch 1, worin R Methylthioäthyl ist.
20. Zusammensetzungen nach Anspruch 1, worin R Äthoxyäthyl ist.
21. Zusammensetzungen nach Anspruch 1, worin R Äthyl ist.
22. Verfahren zum Bekämpfen von unerwünschten Pflanzen, welches das Aufbringen eines herbizid wirksamen Anteils einer Verbindung der Formel ausgewählt aus

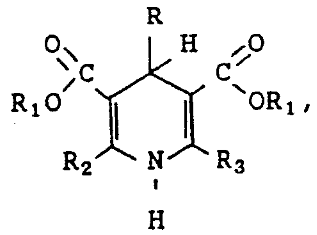

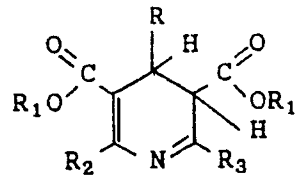

und

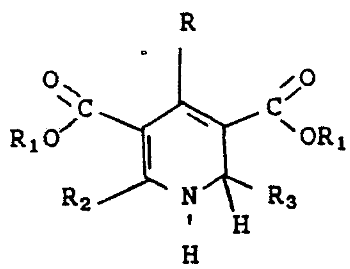

worin R ausgewählt ist aus Phenyl-, $C_{1-6}$-Alkyl-, $C_{1-6}$-Halogenalkyl-, $C_{1-6}$-Alkoxy-$C_{1-6}$-alkyl, $C_{1-6}$-Alkylthio-$C_{1-6}$-alkyl-, $C_{1-6}$-Alkylcarbonyloxy-$C_{1-6}$-alkyl, $C_{3-6}$-Cycloalkyl-, $C_{3-6}$-Cycloalkyl-$C_{1-6}$-alkyl, Phenylmethoxymethyl, Phenoxy-$C_{1-6}$-alkyl, Pyridyl, Furyl- und Thienylresten; jedes $R_1$ unabhängig ausgewählt ist aus $C_{1-4}$-Alkylresten und $R_2$ und $R_3$ unabhängig ausgewählt sind aus $C_{1-6}$-Alkyl- und fluorierten Methylresten, vorausgesetzt, daß mindestens eines von $R_2$ und $R_3$ fluoriertes Methyl sein muß, an deren Ort umfaßt.

23. Verfahren nach Anspruch 22, worin jedes von $R_2$ und $R_3$ unabhängig ausgewählt ist aus fluorierten Methylresten.
24. Verfahren nach Anspruch 23, worin R $C_{1-6}$-Alkyl ist.
25. Verfahren nach Anspruch 24, worin eines von $R_2$ und $R_2$ ein Trifluormethylrest ist.
26. Verfahren nach Anspruch 24, worin eines von $R_2$ und $R_3$ ein Difluormethylrest ist.
27. Verfahren nach Anspruch 22, worin R ausgewählt ist aus $C_{2-4}$-Alkyl und Cyclopropylmethyl.
28. Verfahren nach Anspruch 27, worin $R_1$ $C_{2-4}$-Alkyl ist.
29. Verfahren nach Anspruch 28, worin eines von $R_2$ und $R_3$ ein Trifluormethylrest ist.
30. Verfahren nach Anspruch 28, worin eines von $R_2$ und $R_3$ ein Difluormethylrest ist.
31. Verfahren nach Anspruch 28, worin $R_2$ fluoriertes Alkyl und $R_3$ Alkyl sind.
32. Verfahren nach Anspruch 22, worin R Isobutyl ist.
33. Verfahren nach Anspruch 32, worin $R_1$ Methyl ist.
34. Verfahren nach Anspruch 33, worin eines von $R_2$ und $R_3$ ein Trifluormethylrest ist.
35. Verfahren nach Anspruch 33, worin eines von $R_2$ und $R_3$ ein Difluormethylrest ist.
36. Verfahren nach Anspruch 22, worin R Furyl ist.
37. Verfahren nach Anspruch 22, worin R Pyridyl ist.
38. Verfahren nach Anspruch 22, worin R Thienyl ist.
39. Verfahren nach Anspruch 23, worin R Methylthiomethyl ist.
40. Verfahren nach Anspruch 23, worin R Methoxymethyl ist.
41. Verfahren nach Anspruch 23, worin R Methylthioäthyl ist.
42. Verfahren nach Anspruch 23, worin R Äthoxyäthyl ist.
43. Verfahren nach Anspruch 1, worin R Äthyl ist.
44. Verbindung der Formel ausgewählt aus

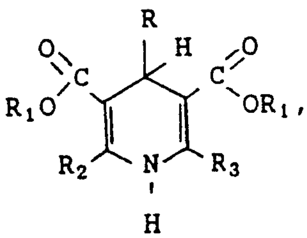
,
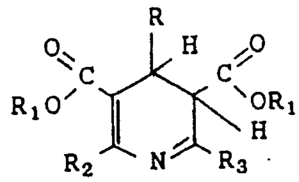
und

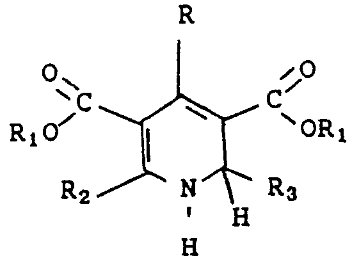

worin R ausgewählt ist aus Phenyl-, $C_{1-6}$-Alkyl-, $C_{1-6}$-Halogenalkyl-, $C_{1-6}$-Hydroxyalkyl-, $C_{1-6}$-Alkoxy-$C_{1-6}$-alkyl, $C_{1-6}$-Alkylthio-$C_{1-6}$-alkyl-, $C_{1-6}$-Alkylcarbonyloxy-$C_{1-6}$-alkyl, $C_{3-6}$-Cycloalkyl-, $C_{3-6}$-Cycloalkyl-$C_{1-6}$-alkyl, Phenylmethoxymethyl, Phenoxy-$C_{1-6}$-alkyl, Pyridyl, Furyl- und Thienylresten; jedes $R_1$ unabhängig ausgewählt ist aus $C_{1-4}$-Alkylresten und $R_2$ und $R_3$ unabhängig ausgewählt sind aus $C_{1-6}$-Alkyl- und fluorierten Methylresten, vorausgesetzt, daß mindestens eines von $R_2$ und $R_3$ fluoriertes Methyl sein muß.

45. Verbindungen nach Anspruch 44, worin R $C_{1-6}$-Alkyl und $R_1$ Äthyl sind.

46. Verbindungen nach Anspruch 44, worin R $C_{1-6}$-Alkyl und $R_1$ Methyl sind.

47. Verbindungen nach Anspruch 44, worin eines von $R_2$ und $R_3$ ein Trifluormethylrest ist.

48. Verbindungen nach Anspruch 44, worin eines von $R_2$ und $R_3$ ein Difluormethylrest ist.

49. Verbindungen nach Anspruch 44, worin R ausgewahlt ist aus $C_{2-4}$-Alkyl und Cyclopropylmethyl.

50. Verbindungen nach Anspruch 49, worin eines von $R_2$ und $R_3$ ein Trifluormethylrest ist.

51. Verbindungen nach Anspruch 49, worin eines von $R_2$ und $R_3$ ein Difluormethylrest ist.

52. Verbindungen nach Anspruch 49, worin $R_2$ fluoriertes Methyl und $R_3$ Alkyl sind.

53. Verbindungen nach Anspruch 44, worin R Isobutyl ist.

54. Verbindungen nach Anspruch 49, worin $R_1$ ausgewählt ist aus Methyl und Äthyl.

55. Verbindungen nach Anspruch 54, worin R Furyl ist.

56. Verbindungen nach Anspruch 44, worin R Pyridyl ist.

57. Verbindungen nach Anspruch 44, worin R Thienyl ist.

58. Verbindungen nach Anspruch 44, worin R ein $C_{1-6}$-Alkylthio-$C_{1-6}$-alkylrest ist.

59. Verbindungen nach Anspruch 58, worin R ein Methylthiomethylrest ist.

60. Verbindungen nach Anspruch 58, worin R ein Methylthioäthylrest ist.

61. Verbindungen nach Anspruch 44, worin R Halogen-$C_{1-6}$-alkyl ist.

62. Verbindungen nach Anspruch 61, worin R ausgewählt ist aus Chlormethyl, Trifluormethyl und 2-(Trifluormethyl)-propyl.

63. Verbindungen nach Anspruch 44, worin R ein $C_{1-6}$-Alkylcarbonyloxy-$C_{1-6}$-alkylrest ist.

64. Verbindungen nach Anspruch 63, worin R Acetoxymethyl ist.

65. Verbindungen nach Anspruch 44, worin R ein $C_{1-6}$-Alkoxy-$C_{1-6}$-alkylrest ist.

66. Verbindungen nach Anspruch 65, worin R Methoxymethyl ist.

67. Verbindungen nach Anspruch 65, worin R Äthoxyäthyl ist.

68. Verfahren zum Herstellen von Dihydropyridinen einer Formel ausgewählt aus

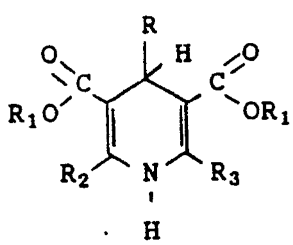
und
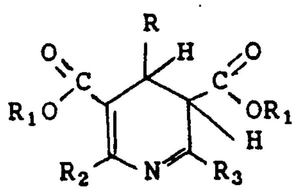

worin R ausgewählt ist aus Phenyl-, $C_{1-6}$-Alkyl-, $C_{1-6}$-Halogenalkyl-, $C_{1-6}$-Hyroxyalkyl-, $C_{1-6}$-Alkoxy-$C_{1-6}$-alkyl, $C_{1-6}$-Alkylthio-$C_{1-6}$-alkyl-, $C_{1-6}$-Alkylcarbonyloxy-$C_{1-6}$-alkyl, $C_{3-6}$-Cycloalkyl-, $C_{3-6}$-Cycloalkyl-$C_{1-6}$-alkyl, Phenylmethoxymethyl, Phenoxy-$C_{1-6}$-alkyl, Pyridyl, Furyl- und Thienylresten; jedes $R_1$ unabhängig ausgewählt ist aus $C_{1-4}$-Alkylresten und $R_2$ und $R_3$ die gleichen fluorierten Methylreste sind, in einer einzigen Reaktionsmischung, welches die Schritte des

(1) Umsetzens eines Alkyl-3-ketoesters der Formel

$R_2\overset{O}{\overset{\|}{C}}CH_2\overset{O}{\overset{\|}{C}}OR_1$ und $R_3\overset{O}{\overset{\|}{C}}-CH_2\overset{O}{\overset{\|}{C}}-OR_1$

worin $R_1$, $R_2$ und $R_3$ wie oben definiert sind, mit einem Aldehyd der Formel

$$R\overset{O}{\overset{\|}{C}}H$$

worin R wie oben definiert ist;

(2) Zusetzens eines Reaktanten ausgewählt aus Ammoniumhydroxid und gasförmigem Ammoniak zum Reaktionsprodukt von Schritt (1) in Anwesenheit eines aprotischen inerten Lösungsmittels zum Vorsehen eines Dihydroxypiperidins und

(3) Dehydratisierens dieses Dihydroxpiperidins mit einem Dehydratisierungsmittel zum Vorsehen des genannten Dihydropyridins umfaßt.

69. Verfahren nach Anspruch 68, worin der Alkyl-3-ketoester Äthyltrifluoracetoacetat ist.

70. Verfahren nach Anspruch 68, worin der Alkyl-3-ketoester Äthyldifluoracetoacetat und Methyltrifluoracetoacetat ist.

71. Verfahren nach Anspruch 68, worin der Aldehyd Propionaldehyd ist.

72. Verfahren nach Anspruch 68, worin der Aldehyd Butyraldehyd ist.

73. Verfahren nach Anspruch 68, worin der Aldehyd Isovaleraldehyd ist.

**Revendications**

1. Compositions herbicides comprenant un adjuvant et une quantité efficace comme herbicide d'un composé répondant à une formule choisie parmi:

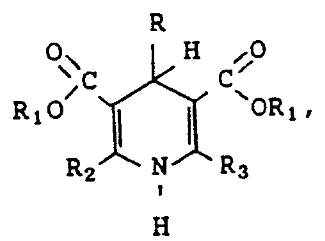,
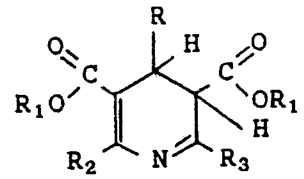
et
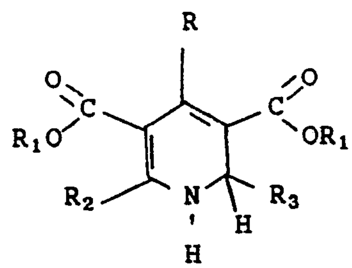

dans laquelle R est choisi parmi des radicaux phényle, alkyle en $C_1$ à $C_6$, haloalkyle en $C_1$ à $C_6$, (alcoxy en $C_1$ à $C_6$) (alkyle en $C_1$ à $C_6$), (alkyle en $C_1$ à $C_6$)thio(alkyle en $C_1$ à $C_6$), (alkyle en $C_1$ à $C_6$)carbonyloxy (alkyle en $C_1$ à $C_6$, cycloalkyle en $C_3$ à $C_6$, (cycloalkyle en $C_3$ à $C_6$) (alkyle en $C_1$ à $C_6$), phénylméthoxyméthyle, phénoxy-(alkyle en $C_1$ à $C_6$), pyridyle, furyle et thiényle; chacun des $R_1$ est choisi indépendamment parmi des radicaux alkyle en $C_1$ à $C_4$ et $R_2$ et $R_3$ sont choisis indépendamment parmi des radicaux alkyle en $C_1$ à $C_6$ et méthyle fluoré, sous réserve qu'au moins un des symboles $R_2$ et $R_3$ doit être un radical méthyle fluoré.

2. Compositions selon la revendication 1, dans lesquelles R est un radical alkyle en $C_1$ à $C_6$.

3. Compositions selon la revendication 2, dans lesquelles un des symboles $R_2$ et $R_3$ est un radical trifluorométhyle.

4. Compositions selon la revendication 2, dans lesquelles un des radicaux $R_2$ et $R_3$ est un radical difluorométhyle.

5. Compositions selon la revendication 1, dans lesquelles R est choisi parmi un groupe alkyle en $C_2$ à $C_4$ et un groupe cyclopropylméthyle.

6. Compositions selon la revendication 5, dans lesquelles $R_1$ est un radical alkyle en $C_2$ à $C_4$.

7. Compositions selon la revendication 6, dans lesquelles un des symboles $R_2$ et $R_3$ est un radical trifluorométhyle.

8. Compositions selon la revendication 6, dans lesquelles un des symboles $R_2$ et $R_3$ est un radical difluorométhyle.

9. Compositions selon la revendication 6, dans lesquelles $R_2$ est un radical fluoralkyle et $R_3$ est un radical alkyle.

10. Compositions selon la revendication 1, dans lesquelles R est un radical isobutyle.

11. Compositions selon la revendication 10, dans lesquelles $R_1$ est un radical méthyle.

12. Compositions selon la revendication 11, dans lesquelles un des symboles $R_2$ et $R_3$ est un radical trifluorométhyle.

13. Compositions selon la revendication 11, dans lesquelles un des symboles $R_2$ et $R_3$ est un radical difluorométhyle.

14. Compositions selon la revendication 1, dans lesquelles R est un radical furyle.

15. Compositions selon la revendication 1, dans lesquelles R est un radical pyridyle.

16. Compositions selon la revendication 1, dans lesquelles R est un radical thiényle.

17. Compositions selon la revendication 1, dans lesquelles R est un radical méthylthiométhyle.

18. Compositions selon la revendication 1, dans lesquelles R est un radical méthoxyméthyle.

19. Compositions selon la revendication 1, dans lesquelles R est un radical méthylthioéthyle.

20. Compositions selon la revendication 1, dans lesquelles R est un radical éthoxyéthyle.

21. Compositions selon la revendication 1, dans lesquelles R est un radical éthyle.

22. Procédé pour lutter contre les plantes indésirables, qui comprend l'application, sur l'emplacement de celles-ci, d'une quantité efficace comme herbicide d'un composé répondant à une formule choisie parmi:

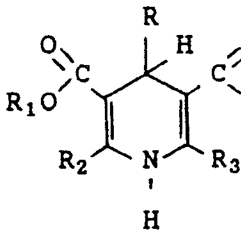

,

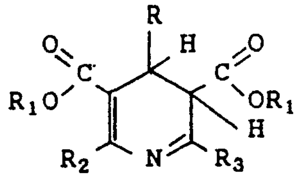

et

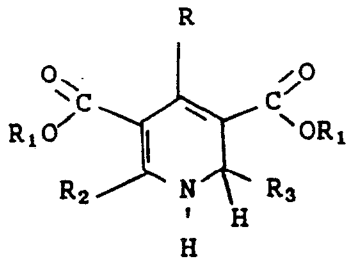

dans laquelle R est choisi parmi des radicaux phényle, alkyle en $C_1$ à $C_6$, haloalkyle en $C_1$ à $C_6$, (alcoxy en $C_1$ à $C_6$) (alkyle en $C_1$ à $C_6$), (alkyle en $C_1$ à $C_6$)thio(alkyle en $C_1$ à $C_6$), (alkyle en $C_1$ à $C_6$)carbonyloxy (alkyle en $C_1$ à $C_6$), cycloalkyle en $C_3$ à $C_6$, (cycloalkyle en $C_3$ à $C_6$) (alkyle en $C_1$ à $C_6$), phénylméthoxyméthyle, phénoxy-(alkyle en $C_1$ à $C_6$), pyridyle, furyle et thiényle; chacun des $R_1$ est choisi indépendamment parmi des radicaux alkyle en $C_1$ à $C_4$ et $R_2$ et $R_3$ sont choisi indépendamment parmi des radicaux alkyle en $C_1$ à $C_6$ et méthyle fluoré, sous réserve qu'au moins un des symboles $R_2$ et $R_3$ doit être un radical méthyle fluoré.

23. Procédé selon la revendication 22, dans lequel chacun des symboles $R_2$ et $R_3$ est choisi indépendamment parmi les radicaux méthyle fluorés.

24. Procédé selon la revendication 23, dans lequel R est un radical alkyle en $C_1$ à $C_6$.

25. Procédé selon la revendication 24, dans lequel un des symboles $R_2$ et $R_3$ est un radical trifluorométhyle.

26. Procédé selon la revendication 24, dans lequel un des symboles $R_2$ et $R_3$ est un radical difluorométhyle.

27. Procédé selon la revendication 22, dans lequel R est choisi parmi des radicaux alkyle en $C_2$ à $C_4$ et cyclopropylméthyle.

28. Procédé selon la revendication 27, dans lequel $R_1$ est un radical alkyle en $C_2$ à $C_4$.

29. Procédé selon la revendication 28, dans lequel un des symboles $R_2$ et $R_3$ est un radical trifluorométhyle.

30. Procédé selon la revendication 28, dans lequel un des symboles $R_2$ et $R_3$ est un radical difluorométhyle.

31. Procédé selon la revendication 28 dans lequel $R_2$ est un radical méthyle fluoré et $R_3$ est un radical alkyle.

32. Procédé selon la revendication 22, dans lequel R est un radical isobutyle.

33. Procédé selon la revendication 32, dans lequel $R_1$ est un radical méthyle.

34. Procédé selon la revendication 33, dans lequel un des symboles $R_2$ et $R_3$ est un radical trifluorométhyle.

35. Procédé selon la revendication 33, dans lequel un des symboles $R_2$ et $R_3$ est un radical difluorométhyle.

36. Procédé selon la revendication 22, dans lequel R est un radical furyle.
37. Procédé selon la revendication 22, dans lequel R est un radical pyridyle.
38. Procédé selon la revendication 22, dans lequel R est un radical thiényle.
39. Procédé selon la revendication 23, dans lequel R est un radical méthylthiométhyle.
40. Procédé selon la revendication 23, dans lequel R est un radical méthoxyméthyle.
41. Procédé selon la revendication 23, dans lequel R est un radical méthylthioéthyle.
42. Procédé selon la revendication 23, dans lequel R est un radical éthoxyéthyle.
43. Procédé selon la revendication 23, dans lequel R est un radical éthyle.
44. Composés répondant aux formules choisies parmi:

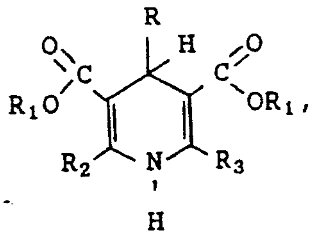
,
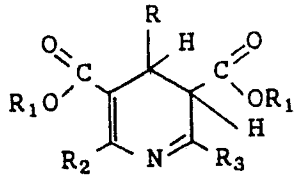
et

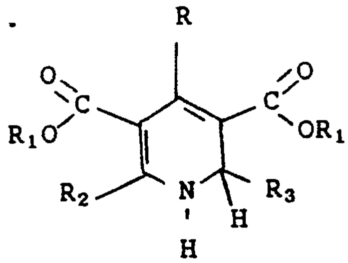

dans lesquelles R est choisi parmi des radicaux phényle, alkyle en $C_1$ à $C_6$, haloalkyle en $C_1$ à $C_6$, hydroxylalkyle en $C_1$ à $C_6$, (alcoxy en $C_1$ à $C_6$) (alkyle en $C_1$ à $C_6$), (alkyle en $C_1$ à $C_6$)thio(alkyle en $C_1$ à $C_6$), (alkyle en $C_1$ à $C_6$)carbonyloxy (alkyle en $C_1$ à $C_6$), cycloalkyle en $C_3$ à $C_6$, (cycloalkyle en $C_3$ à $C_6$) (alkyle en $C_1$ à $C_6$), phénylméthoxyméthyle, phénoxy(alkyle en $C_1$ à $C_6$), pyridyle, furyle et thiényle; chacun des $R_1$ est choisi indépendamment parmi des radicaux alkyle en $C_1$ à $C_4$ et $R_2$ et $R_3$ sont choisis indépendamment parmi des radicaux alkyle en $C_1$ à $C_6$ et méthylefluoré, sous réserve qu'au moins un des symboles $R_2$ et $R_3$ doit être un radical méthyle fluoré.

45. Composés selon la revendication 44, dans lesquels R est un radical alkyle en $C_1$ à $C_6$ et $R_1$ un radical éthyle.

46. Composés selon la revendication 44, dans lesquels R est un radical en $C_1$ à $C_6$ et $R_1$ est un radical méthyle.

47. Composés selon la revendication 44, dans lesquels un des symboles $R_2$ et $R_3$ est un radical trifluorométhyle.

48. Composés selon la revendication 44, dans lesquels un des symboles $R_2$ et $R_3$ est un radical difluorométhyle.

49. Composés selon la revendication 44, dans lesquels R est choisi parmi un radical alkyle en $C_2$ à $C_4$ et un radical cyclopropylméthyle.

50. Composés selon la revendication 49, dans lesquels un des symboles $R_2$ et $R_3$ est un radical trifluorométhyle.

51. Composés selon la revendication 49, dans lesquels un des symboles $R_2$ et $R_3$ est un radical difluorométhyle.

52. Composés selon la revendication 49, dans lesquels $R_2$ est un radical méthyle fluoré et $R_3$ est un radical alkyle.

53. Composés selon la revendication 44, dans lesquels R est un radical isobutyle.

54. Composés selon la revendication 49, dans lesquels $R_1$ est choisi parmi les radicaux méthyle et éthyle.

55. Composés selon la revendication 54, dans lesquels R est un radical furyle.

56. Composés selon la revendication 44, dans lesquels R est un radical pyridyle.

57. Composés selon la revendication 44, dans lesquels R est un radical thiényle.

58. Composés selon la revendication 44, dans lesquels R est un radical (alkyle en $C_1$ à $C_6$)thio(alkyle en $C_1$ à $C_6$).

59. Composés selon la revendication 58, dans lesquels R est un radical méthylthiométhyle.

60. Composés selon la revendication 58, dans lesquels R est un radical méthylthioéthyle.

61. Composés selon la revendication 44, dans lesquels R est un radical halo(alkyle en $C_1$ à $C_6$).

62. Composés selon la revendication 61, dans lesquels R est choisi parmi les radicaux chlorométhyle, trifluorométhyle et 2-(trifluorométhyl)propyle.

63. Composés selon la revendication 44, dans lesquels R est un radical (alkyle en $C_1$ à $C_6$)carbonyloxy (alkyle en $C_1$ à $C_6$).

64. Composés selon la revendication 63, dans lesquels R est un radical acétoxyméthyle.

65. Composés selon la revendication 44, dans lesquels R est un radical (alcoxy en $C_1$ à $C_6$) (alkyle en $C_1$ à $C_6$).

66. Composés selon la revendication 65, dans lesquels R est un radical méthoxyméthyle.

67. Composés selon la revendication 65, dans lesquels R est un radical éthoxyéthyle.

68. Procédé de préparation de dihydropyridines répondant aux formules choisies parmi:

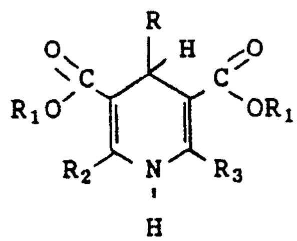

et

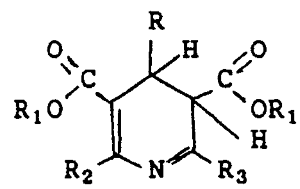

dans lesquelles R est choisi parmi des radicaux phényle, alkyle en $C_1$ à $C_6$, haloalkyle en $C_1$ à $C_6$, hydroxyalkyle en $C_1$ à $C_6$, (alcoxy en $C_1$ à $C_6$) (alkyle en $C_1$ à $C_6$), (alkyle en $C_1$ à $C_6$)thio(alkyle en $C_1$ à $C_6$), (alkyle en $C_1$ à $C_6$)carbonyloxy (alkyle en $C_1$ à $C_6$, cycloalkyle en $C_3$ à $C_6$, (cycloalkyle en $C_3$ à $C_6$) (alkyle en $C_1$ à $C_6$), phénylméthoxyméthyle, phénoxy(alkyle en $C_1$ à $C_6$), pyridyle, furyle et thiényle; chaque $R_1$ est choisi indépendamment parmi les radicaux alkyle en $C_1$ à $C_4$ et $R_2$ et $R_3$ sont les mêmes radicaux méthyle fluorés dans un mélange réactionnel unique qui comprend les stades consistant à:

(1) faire réagir un 3-cétoester alkylique représenté par les formules:

$$R_2\overset{O}{\overset{\|}{C}}CH_2\overset{O}{\overset{\|}{C}}OR_1 \quad \text{et} \quad R_3\overset{O}{\overset{\|}{C}}-CH_2\overset{O}{\overset{\|}{C}}-OR_1$$

dans lesquelles $R_1$, $R_2$ et $R_3$ sont tels que définis ci-dessus, avec un aldéhyde représenté par la formule:

$$R\overset{O}{\overset{\|}{C}}H$$

dans laquelle R est tel que défini ci-dessus;

(2) ajouter un réactif choisi parmi l'hydroxyde d'ammonium et l'ammoniac à travers le produit de réaction du stade (1) en présence d'un solvant inerte aprotique pour donner une dihydroxypipéridine et

(3) déshydration cette dihydroxypipéridine avec un agent de déshydratation pour donner cette dihydropyridine.

69. Procédé selon la revendication 68, dans lequel le 3-cétoester alkylique est le trifluoracétoacétate d'éthyle.

70. Procédé selon la revendication 68, dans lequel le 3-cétoester alkylique est le difluoroacétoacétate d'éthyle et le trifluoroacétoacétate de méthyle.

71. Procédé selon la revendication 68, dans lequel l'aldéhyde est le propionaldéhyde.

72. Procédé selon la revendication 68, dans lequel l'aldéhyde est le butyraldéhyde.

73. Procédé selon la revendication 68, dans lequel l'aldéhyde est l'isovaléraldéhyde.